# EUROPEAN PATENT APPLICATION

(11) **EP 4 570 835 A1**
(43) Date of publication of application: **18.06.2025**
(21) Application number: 23852645.3
(22) Date of filing: 10.08.2023
(51) Int. Cl.: C08F 220/12, A61K 9/51, A61K 47/32, A61K 47/58, A61K 47/66, A61P 35/00, C08F 290/06

(54) **NOVEL COPOLYMER**

(30) Priority: 10.08.2022 JP 2022128535
(71) Applicant: KOWA COMPANY, LTD., Naka-ku Nagoya-shi Aichi 460-8625 (JP)
(72) Inventor: SUZUKI, Kenichi, Higashimurayama-shi, Tokyo 189-0022 (JP); FUJIMAKI, Nobuhiro, Higashimurayama-shi, Tokyo 189-0022 (JP); ARAI, Yohei, Higashimurayama-shi, Tokyo 189-0022 (JP)
(74) Representative: Wächtershäuser & Hartz Patentanwaltspartnerschaft mbB
(86) International application number: PCT/JP2023/029355
(87) International publication number: WO 2024/034683

(57) **Abstract**

To provide a novel copolymer utilizable for drug delivery technology.

The present invention relates to a copolymer in which a target-affinity molecule is bonded to a copolymer X having structural units of (A), (B), and (C). 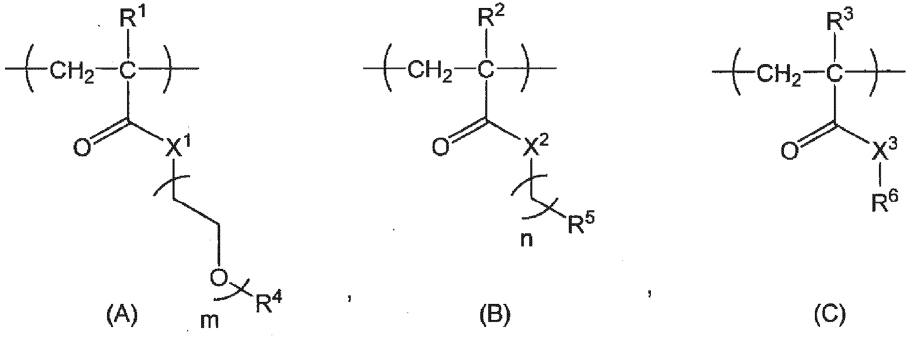 R¹, R², and R³ are the same or different and represent hydrogen or C₁₋₃ alkyl; R⁴ represents C₁₋₃ alkyl; R⁵ represents hydrogen, C₁₋₁₈ alkyl, 3- to 8- membered cycloalkyl optionally having substituent, adamantyl, C₆₋₁₈ aryl optionally having substituent, or a 5- to 10- membered heteroaryl optionally having substituent; X¹, X², and X³ are the same or different and represent oxygen, sulfur, or N-R⁷; R⁶ represents hydrogen, leaving group, or linker; R⁷ represents hydrogen or C₁₋₃ alkyl; m represents 1 to 100; and n represents 0 to 3.

## Description

### Technical Field

The present invention relates to a novel copolymer useful for drug delivery technology. More specifically, the present invention relates to a copolymer for a drug delivery carrier targeting tumors, a pharmaceutical composition in which a physiologically active substance such as an anticancer agent is carried on the copolymer, and a medicine containing the composition.

### Background Art

In recent years, research on drug delivery systems (DDS) has been energetically conducted as a technique for efficiently and safely delivering drugs to disease sites. Among them, there is an increasing demand for DDS employing nanoparticles as drug delivery carriers as a technique for enhancing the selectivity of drug accumulation by utilizing the structural characteristics of the disease site.

For example, in solid cancer tissue, since the structure of a neovascular vessel (tumor blood vessel) is immature as compared with a normal blood vessel, a cell gap of about several hundred nm is generated in the vascular endothelium, which allows the permeation of a large amount of substance. Due to this structural feature, it is known that a polymeric compound containing nanoparticles selectively permeates a tumor blood vessel and accumulates in solid cancer tissue. Furthermore, in solid cancer tissue, a lymphatic system involved in excretion of polymers malfunctions, so that permeated nanoparticles are continuously retained in the tissue (Enhanced permeability and retention effect, EPR effect). Since a general low molecular drug leaks out of a blood vessel due to membrane permeation of vascular cells, it is non-selectively distributed in tissue and does not accumulate in solid cancer tissue. According to the methodology of the EPR effect, nanoparticle-based drug delivery results in improved tissue selectivity to solid cancer in the distribution of a drug, since distribution to tissue is governed by the permeability of vascular endothelial cell gaps. Therefore, the EPR effect is a promising academic support in the development of nanotechnology-applied medicines (nanomedicine) targeting solid cancer.

It is believed that a drug delivery process in the EPR effect occurs through blood flow and that the extravasation process of nanoparticles is passive. Therefore, to maximize the accumulation of nanoparticles in solid cancer, it is important to impart a molecular design that can withstand long-term blood retention to the constituent components of nanoparticles as drug delivery carriers. Drug delivery carriers are therefore required to have the ability to avoid barriers such as non-specific interactions with blood components, foreign body recognition by the reticuloendothelial system (RES) in the liver, spleen, and lungs, and glomerular filtration in the kidneys. In addition, it is known that these barriers can be overcome by optimizing particle properties such as particle diameter and surface modification with a biocompatible polymer. For example, it is desirable that the particle diameter of the drug delivery carrier be greater than about 6 nm, which is the threshold for renal clearance, and less than 200 nm, which may avoid recognition by the RES.

The particle diameter of the drug delivery carrier is also known to affect tissue permeation at a disease site. For example, the anticancer activity of drug-encapsulating nanoparticles with particle diameters measuring 30 nm, 50 nm, 70 nm, and 100 nm, which exhibit equivalent blood retention, has been compared and studied, and it has been revealed that drug-encapsulating nanoparticles with a particle diameter of 30 nm reach the deep parts of a disease site and thus exhibit the highest therapeutic effect (Non-Patent Literature 1). Therefore, it would be desirable for the particle diameter of nanoparticles for a drug delivery carrier targeting solid cancer to be as small as possible within the range that avoids renal clearance.

As nanoparticles for drug delivery carriers, methods using colloidal dispersions such as liposomes, emulsions, or nanoparticles, methods using biologically derived raw materials such as albumin, methods using natural polymers such as natural polysaccharides, or methods using synthetic polymers, have been developed. Among them, synthetic polymers are widely used as constituents of drug delivery carriers because it is possible to prepare nanoparticles whose particle diameter is precisely controlled by appropriately selecting monomers as constituent components and synthesis methods.

For example, a method for utilizing an amphiphilic block copolymer composed of a hydrophilic segment and a hydrophobic segment as a drug delivery carrier is disclosed. The block copolymer spontaneously associates in an aqueous medium by, for example, hydrophobic interaction between molecules as a driving force to form core-shell type nanoparticles (polymeric micelles). It is known that it is possible to encapsulate a low molecular drug in or bind it to the hydrophobic segment of the polymeric micelle, and the obtained drug-encapsulating polymeric micelle exhibits high blood stability, and due to selective accumulation in solid cancer through the EPR effect, high anticancer activity as compared with the administration of a solution of a low molecular drug (Patent Literature 1). However, since the polymeric micelle is an assembly of multiple molecules, a particle diameter of about 30 nm is the lower limit value that can be prepared, and it is difficult to finely control the size in the vicinity of a particle diameter of 10 nm that can avoid the influence of renal clearance.

Meanwhile, among nanoparticles formed of a synthetic polymer, those which form particles by, for example, chemical cross-linking, hydrophobic interaction, or ionic bonding within a single chain as a driving force (hereinafter abbreviated as single chain nanoparticles (SCNPs)) are known to form nanoparticles having a small particle diameter of 20 nm or less (Non-Patent Literature 2). Therefore, although SCNPs are expected to be useful as drug delivery carriers, a technique for precisely controlling their particle diameter has not been found so far.

Another is a drug delivery system that utilizes avidin/biotin binding. Affinity between avidin/streptavidin and biotin is very high, and their interaction is widely applied in the fields of biochemistry, molecular biology, and medical science (Non-Patent Literatures 3 and 4). Moreover, drug delivery methods and pre-targeting methods combining this high binding capacity with antibody molecules have been devised (Non-Patent Literature 5).

### Citation List

### Patent Literature

Patent Literature 1: JP 3270592 B

### Non-Patent Literature

Non-Patent Literature 1: H. Cabral et al., Nat. Nanotechnol. 6 815-823 (2011)
Non-Patent Literature 2: Jose A. Pomposo, Single-Chain Polymer Nanoparticles: Synthesis, Characterization, Simulations, and Applications (2017)
Non-Patent Literature 3: N. S. Green, Adv. Protein Chem., 29:85-133 (1975)
Non-Patent Literature 4: N. S. Green, Methods Enzymol. 184:51-67 (1990)
Non-Patent Literature 5: D. J. Hnatowich et al., J. Nucl. Med. 28, 1294-1302 (1987)

### Summary of Invention

### Technical Problem

It is an object of the present invention to provide a copolymer for a drug delivery carrier targeting a tumor. More specifically, it is an object of the present invention to provide a copolymer for a drug delivery carrier designed for improving blood retention and/or tumor accumulation of a drug.

### Solution to Problem

In order to achieve the above-mentioned objects, the present inventors energetically studied, and found a property that a terpolymer of an acrylic acid derivative forms the SCNP in water. In addition, the present inventors succeeded in creating a novel polymer for a drug delivery carrier, which is capable of precisely controlling a particle diameter of an SCNP at a minute scale of 20 nm or less and about 10 nm, and has high tumor accumulation. As an anticancer agent was carried on the polymer and administered to a mouse model subcutaneously implanted with a colon cancer, an excellent antitumor effect was exhibited. Moreover, the present inventors also succeeded in creating the terpolymer derivative that forms a biotin-bonded SCNP applicable to the pre-targeting methods.

The present invention relates to the following inventions.
[1] A copolymer in which a target-affinity molecule is bonded to a copolymer X comprising structural units represented by the following formulas (A), (B), and (C). 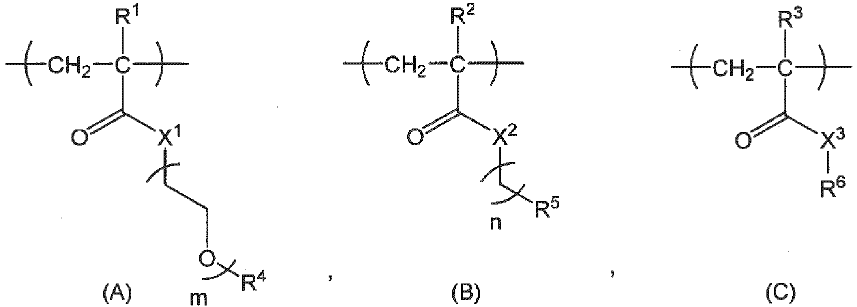 wherein, R¹, R², and R³ are the same or different and represent a hydrogen atom or a C₁₋₃ alkyl group, R⁴ represents a C₁₋₃ alkyl group, R⁵ represents a hydrogen atom, a C₁₋₁₈ alkyl group, a 3- to 8- membered cycloalkyl group optionally having a substituent, an adamantyl group, a C₆₋₁₈ aryl group optionally having a substituent, or a 5-to 10-membered heteroaryl group optionally having a substituent, X¹, X², and X³ are the same or different and represent an oxygen atom, a sulfur atom, or N-R⁷, R⁶ represents a hydrogen atom, a leaving group, or a linker; R⁷ represents a hydrogen atom or a C₁₋₃ alkyl group, m represents an integer of 1 to 100, and n represents an integer of 0 to 3.
[2] The copolymer according to [1], wherein the copolymer X is a copolymer formed by polymerization of three types of monomers represented by the following general formulas (1) to (3), 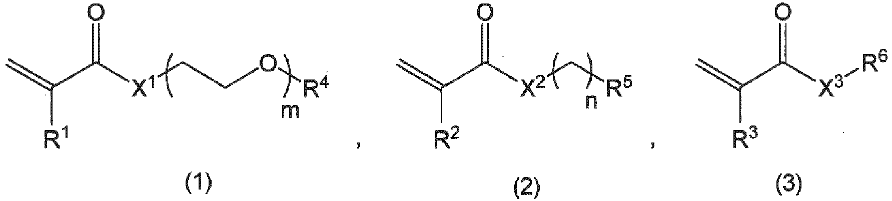 wherein, R¹, R², and R³ are the same or different and represent a hydrogen atom or a C₁₋₃ alkyl group, R⁴ represents a C₁₋₃ alkyl group, R⁵ represents a hydrogen atom, a C₁₋₁₈ alkyl group, a 3- to 8-membered cycloalkyl group optionally having a substituent, an adamantyl group, a C₆₋₁₈ aryl group optionally having a substituent, or a 5-to 10-membered heteroaryl group optionally having a substituent, X¹, X², and X³ are the same or different and represent an oxygen atom, a sulfur atom, or N-R⁷, R⁶ represents a hydrogen atom, a leaving group, or a linker, R⁷ represents a hydrogen atom or a C₁₋₃ alkyl group, m represents an integer of 1 to 100, and n represents an integer of 0 to 3.
[3] The copolymer according to [1] or [2], wherein R¹ is a hydrogen atom.
[4] The copolymer according to any one of [1] to [3], wherein R² is a hydrogen atom.
[5] The copolymer according to any one of [1] to [4], wherein R³ is a hydrogen atom.
[6] The copolymer according to any one of [1] to [5], wherein R⁴ is a methyl group.
[7] The copolymer according to any one of [1] to [6], wherein R⁵ is a C₆₋₁₈ aryl group optionally having a substituent.
[8] The copolymer according to any one of [1] to [7], wherein R⁵ is a phenyl group.
[9] The copolymer according to any one of [1] to [8], wherein R⁶ is a hydrogen atom.
[10] The copolymer according to any one of [1] to [8], wherein the leaving group of R⁶ is represented by the following formula (4). 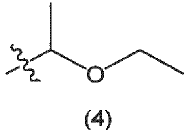
[11] The copolymer according to any one of [1] to [8], wherein the linker of R⁶ is one selected from the following formulas (5) to (7). 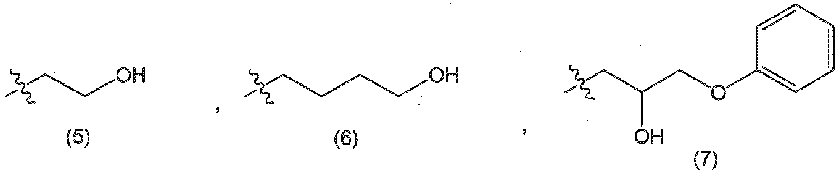
[12] The copolymer according to any one of [1] to [11], wherein X¹ is an oxygen atom.
[13] The copolymer according to any one of [1] to [12], wherein X² is an oxygen atom.
[14] The copolymer according to any one of [1] to [13], wherein X³ is an oxygen atom.
[15] The copolymer according to any one of [1] to [14], wherein m is an integer of 4 to 22.
[16] The copolymer according to any one of [1] to [15], wherein n is 1.
[17] The copolymer according to any one of [1] to [16], wherein a ratio of structural units (A), (B), and (C) is composed of 0.01 to 100 parts by mass of (B) and 0.1 to 100 parts by mass of (C) with respect to 1 part by mass of (A).
[18] The copolymer according to any one of [2] to [16], wherein 0.01 to 100 parts by mass of monomer (2) and 0.1 to 100 parts by mass of monomer (3) are polymerized with respect to 1 part by mass of monomer (1).
[19] The copolymer according to any one of [1] to [18], wherein the copolymer has a number average molecular weight of 5 000 to 150 000.
[20] The copolymer according to any one of [1] to [19], wherein the target-affinity molecule is biotin or a derivative thereof.
[21] The copolymer according to any one of [1] to [19], wherein the target-affinity molecule is a molecule having a biotin residue represented by the following formula (a): 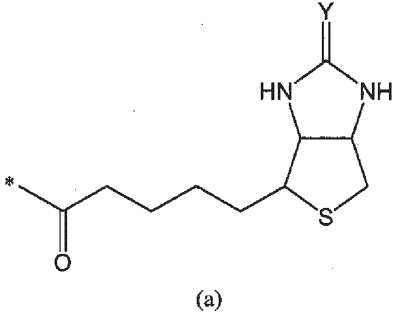 wherein, Y represents O or NH.
[22] The copolymer according to any one of [1] to [19], wherein the target-affinity molecule is any one of molecules represented by the following formulas (8) to (11). 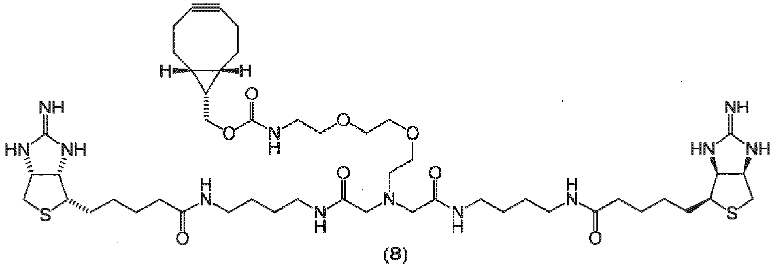 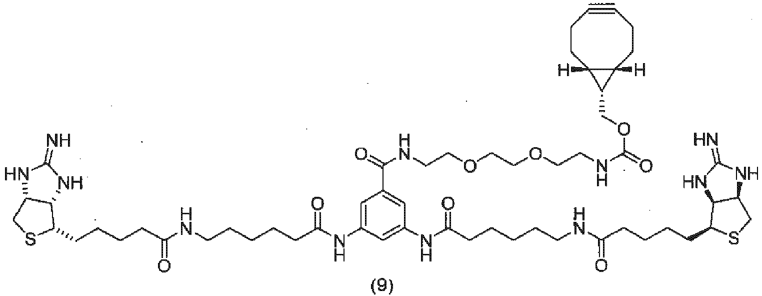 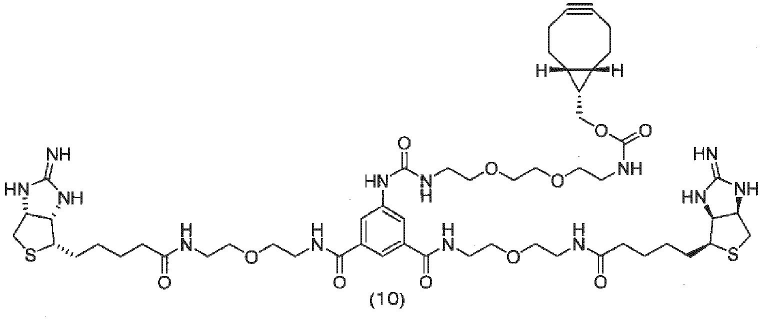 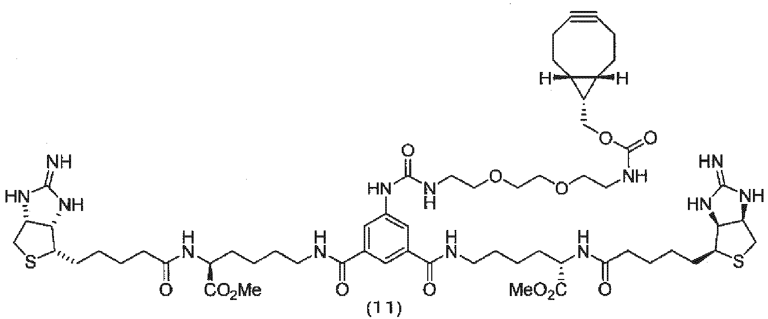
[23] The copolymer according to any one of [1] to [22], wherein the bond of the target-affinity molecule to the copolymer X is a covalent bond or a non-covalent bond.
[24] A single chain nanoparticle including the copolymer according to any one of [1] to [23].
[25] A pharmaceutical composition including the copolymer according to any one of [1] to [24].
[26] The pharmaceutical composition according to [25], for use in a pre-targeting method.

### Advantageous Effects of Invention

As will be evident from Examples described below, an SCNP obtained by self-association of the copolymer of the present invention carrying an anticancer agent, exhibited a tumor growth suppressing in a mouse tumor-bearing model, and therefore can be applied as a therapeutic agent for malignant tumors. The SCNP obtained by the self-association of the copolymer of the present invention, which is carried with the anticancer agent, makes it possible to provide a therapeutic agent for malignant tumors capable of achieving both pharmacological action enhancement and side effect suppression because of having a high effect of suppressing the tumor growth at a low dose. And the copolymer (hereinafter, also referred to as a target-affinity copolymer) to which the target-affinity molecule of the present invention is bonded is useful as a drug delivery and a pre-targeting agent utilizing the biotin/avidin binding.

### Brief Description of Drawings

Fig. 1 is a diagram showing a ¹H-NMR spectrum of a copolymer obtained in Example 1 measured by using nuclear magnetic resonance (NMR).
Fig. 2 is a diagram showing a chromatogram of a copolymer obtained in Example 1 obtained by gel permeation chromatography (GPC).
Fig. 3 is a diagram showing a particle diameter measurement result (scattering intensity distribution) in dynamic light scattering (DLS) for a copolymer before encapsulating DACHPt (Example 69) and a DACHPt-encapsulating SCNP (Example 70).
Fig. 4 is a diagram showing a ¹H-NMR spectrum of a biotin-bonded SCNP obtained in Example 73, measured by using the nuclear magnetic resonance (NMR).
Fig. 5 is a diagram showing a chromatogram of the biotin-bonded SCNP obtained in Example 73 obtained by the gel permeation chromatography (GPC).
Fig. 6 is a diagram showing a particle diameter measurement result (scattering intensity distribution) of the biotin-bonded SCNP obtained in Example 73 in dynamic light scattering (DLS).
Fig. 7 is a diagram showing a ¹H-NMR spectrum of a biotin-bonded SCNP obtained in Example 75, measured by using the nuclear magnetic resonance (NMR).
Fig. 8 is a diagram showing a chromatogram of the biotin-bonded SCNP obtained in Example 75 obtained by the gel permeation chromatography (GPC).
Fig. 9 is a diagram showing a particle diameter measurement result (scattering intensity distribution) of the biotin-bonded SCNP obtained in Example 75 in dynamic light scattering (DLS).
Fig. 10 is a diagram showing a ¹H-NMR spectrum of a biotin-bonded SCNP obtained in Example 77, measured by using the nuclear magnetic resonance (NMR).
Fig. 11 is a diagram showing a chromatogram of the biotin-bonded SCNP obtained in Example 77 obtained by the gel permeation chromatography (GPC).
Fig. 12 is a diagram showing a particle diameter measurement result (scattering intensity distribution) of the biotin-bonded SCNP obtained in Example 77 in dynamic light scattering (DLS).
Fig. 13 is a diagram showing a ¹H-NMR spectrum of a biotin-bonded SCNP obtained in Example 79, measured by using the nuclear magnetic resonance (NMR).
Fig. 14 is a diagram showing a chromatogram of the biotin-bonded SCNP obtained in Example 79 obtained by the gel permeation chromatography (GPC).
Fig. 15 is a diagram showing a particle diameter measurement result (scattering intensity distribution) of the biotin-bonded SCNP obtained in Example 79 in dynamic light scattering (DLS).
Fig. 16 is a diagram showing a change in relative tumor volume when an oxaliplatin solution or the DACHPt-encapsulating SCNP (Example 70) was administered 3 times every other day to a model mouse having a back subcutaneously transplanted with a mouse colon cancer cell line (C26).

### Description of Embodiments

Terms in the present description are used in the meanings commonly used in the field unless otherwise specified. Hereinafter, the present invention will be described in more detail.

In the present description, the term "nanoparticle" refers to a structure showing a particle diameter of 100 nm or less.

In the present description, the term "single chain nanoparticle (SCNP)" refers to a nanoparticle formed by using, for example, chemical crosslinking, hydrophobic interaction, or ionic bonding in a single chain as a driving force. The SCNP often indicates a nanoparticle having a relatively small particle diameter of 20 nm or less among nanoparticles.

In the present description, the term "initiator" means an initiator for thermal radical polymerization such as an azo compound or a peroxide.

In the present description, the term "chain transfer agent" refers to a compound that causes a chain transfer reaction in radical polymerization, and is preferably a compound having a thiocarbonyl group.

In the present description, the term "C₁₋₃ alkyl group" means a linear or branched alkyl group having 1 to 3 carbon atoms, and examples thereof include a methyl group, an ethyl group, an n-propyl group, and an isopropyl group.

In the present description, the term "C₁₋₁₈ alkyl group" means a linear or branched, alkyl group having 1 to 18 carbon atoms, and examples thereof include a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, and an octadecyl group.

In the present description, the term "3- to 8-membered cycloalkyl group optionally having a substituent" means a cyclic alkyl group having 3 to 8 carbon atoms, and examples thereof include a cyclopropyl group, a cyclobutyl group, a cyclopentyl group, a cyclohexyl group, a cycloheptyl group, and a cyclooctyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, the term "C₆₋₁₈ aryl group optionally having a substituent" means a monocyclic or polycyclic condensed aromatic hydrocarbon group, and examples thereof include a phenyl group, a naphthyl group, an anthracenyl group, a phenanthrenyl group, a triphenylenyl group, a pyrenyl group, a chrysenyl group, and a naphthacenyl group. Further, a "C₆₋₁₄ aryl group optionally having a substituent" means a monocyclic or polycyclic condensed aromatic hydrocarbon group, and examples thereof include a phenyl group, a naphthyl group, an anthracenyl group, and a phenanthrenyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, the term "5- to 10-membered heteroaryl group optionally having a substituent" means a 5- to 10-membered, monocyclic aromatic heterocyclic group or condensed aromatic heterocyclic group containing 1 to 4 heteroatoms selected from a nitrogen atom, an oxygen atom, and a sulfur atom, other than a carbon atom, as atoms constituting the ring. Examples of the monocyclic aromatic heterocyclic group include a furyl group, a thienyl group, a pyrrolyl group, a pyridinyl group, a pyrazinyl group, a pyrimidinyl group, a pyridazinyl group, an imidazolyl group, a pyrazyl group, a thiallyl group, an oxazolyl group, an isoxazolyl group, a 1,3,4-thiadiazolyl group, a 1,2,3-triazolyl group, a 1,2,4-triazolyl group, and a tetrazolyl group. Examples of the condensed aromatic heterocyclic group include a benzofuranyl group, a benzothiophenyl group, a quinoxalinyl group, an indolyl group, an isoindolyl group, an isobenzofuranyl group, a chromanyl group, a benzimidazolyl group, a benzothiazolyl group, a benzoxazolyl group, a quinolyl group, and an isoquinolinyl group. The term "6- to 10-membered heteroaryl group optionally having a substituent" means a 6- to 10-membered, monocyclic aromatic heterocyclic group or condensed aromatic heterocyclic group containing 1 to 4 heteroatoms selected from a nitrogen atom, an oxygen atom, and a sulfur atom, other than a carbon atom, as the atoms constituting the ring. Examples of the monocyclic aromatic heterocyclic group include a pyridinyl group, a pyrazinyl group, a pyrimidinyl group, and a pyridazinyl group. Examples of the condensed aromatic heterocyclic group include a benzofuranyl group, a benzothiophenyl group, a quinoxalinyl group, an indolyl group, an isoindolyl group, an isobenzofuranyl group, a chromanyl group, a benzimidazolyl group, a benzothiazolyl group, a benzoxazolyl group, a quinolyl group, and an isoquinolinyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, examples of the "halogen atom" include a fluorine atom, a chlorine atom, a bromine atom, and an iodine atom.

In the present description, the term "target-affinity molecule" means biotin or a derivative thereof having a specific binding function to avidin/streptavidin or a mutant (modified) form or functional fragment thereof. The target-affinity molecule may be carried on the copolymer by an action such as an electrostatic interaction, a hydrogen bond, a hydrophobic interaction, or a covalent bond to the copolymer X of the present invention.

In the present description, the term "pre-targeting method" means a drug delivery method for accurately accumulating a drug on a target cell such as a cancer cell. First, a fusion molecule probe such as a cancer antigen-specific antibody molecule and avidin/streptavidin or a mutant (modified) form or functional fragment thereof is prepared, and administered to a patient to accumulate the fusion molecule on the cancer cell. Next, a process is performed to administer a conjugate of a pharmaceutical composition and a target-affinity molecule such as biotin having affinity for the avidin/streptavidin or the mutant (modified) form or functional fragment thereof to a patient, thereby accurately accumulating the drug on the cancer cell.

It is also possible to prepare a complex in which a conjugate of the molecular probe such as the cancer antigen-specific antibody molecule and the avidin/streptavidin or the mutant (modified) form or functional fragment thereof is bonded to a conjugate of that target-affinity molecule and the pharmaceutical composition in advance and administer the complex to the patient.

In the present description, the term "pharmaceutical composition" means one in which an active ingredient (drug, or physiologically active substance) that can be used for diagnosis, prevention, or treatment of a disease is carried on the copolymer X or the target-affinity copolymer of the present invention by the action such as the electrostatic interaction, the hydrogen bond, the hydrophobic interaction, or the covalent bond. Examples of the carrying form include a form in which the drug is present on the particle surface, a form in which the drug is contained in the nanoparticle, and a combination form thereof where the copolymer X or the target-affinity copolymer forms the nanoparticle.

One embodiment of the present invention is a copolymer (target-affinity copolymer) in which a target-affinity molecule is bonded to a copolymer X comprising structural units represented by the following formulas (A), (B), and (C). 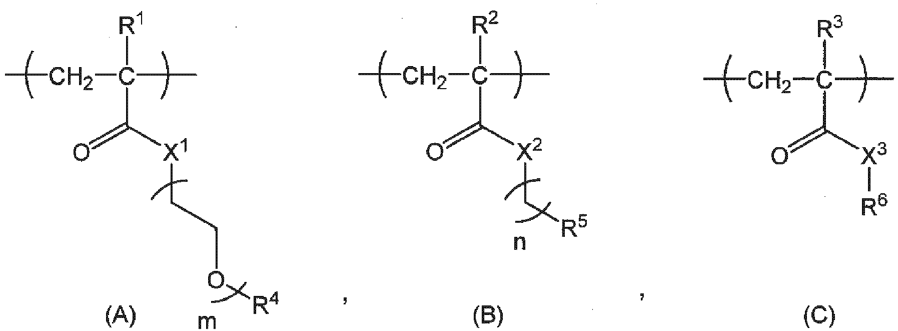 wherein, R¹, R², and R³ are the same or different and represent a hydrogen atom or a C₁₋₃ alkyl group, R⁴ represents a C₁₋₃ alkyl group, R⁵ represents a hydrogen atom, a C₁₋₁₈ alkyl group, a 3- to 8-membered cycloalkyl group optionally having a substituent, an adamantyl group, a C₆₋₁₈ aryl group optionally having a substituent, or a 5-to 10-membered heteroaryl group optionally having a substituent, X¹, X², and X³ are the same or different and represent an oxygen atom, a sulfur atom, or N-R⁷, R⁶ represents a hydrogen atom, a leaving group, or a linker, R⁷ represents a hydrogen atom or a C₁₋₃ alkyl group, m represents an integer of 1 to 100, and n represents an integer of 0 to 3.

In the copolymer X of the present invention, the structural unit (A) functions as a unit that imparts hydrophilicity, and the structural unit (B) functions as a unit that imparts hydrophobicity. Further, the structural unit (C) functions as a scaffold in which the active ingredient (drug, or physiologically active substance) is bonded to the copolymer X or the target-affinity copolymer. Having these three structural units serves to imparting the copolymer X or the target-affinity copolymer a property of forming SCNP in water, and to facilitating the formed SCNP to precisely control particle diameter at a minute scale of 20 nm or less, and to function as a drug delivery carrier having high tumor accumulation.

R¹ in the structural unit (A) represents a hydrogen atom or a C₁₋₃ alkyl group, and is preferably a hydrogen atom or a methyl group, preferably a hydrogen atom, an ethyl group, or a propyl group, and more preferably a hydrogen atom.

X¹ represents an oxygen atom, the sulfur atom, or **N-**R⁷, and is preferably an oxygen atom, a sulfur atom, or NH, and more preferably an oxygen atom.

m represents an integer of 1 to 100, is preferably an integer of 3 to 100, and from a viewpoint of imparting good hydrophilicity, preferably 3 to 80, more preferably 4 to 60, still more preferably 4 to 40, and even more preferably 4 to 22.

R⁴ represents a C₁₋₃ alkyl group, specifically a methyl group, an ethyl group, an n-propyl group or an isopropyl group, preferably a methyl group or an ethyl group, and more preferably a methyl group.

R² in the structural unit (B) represents a hydrogen atom or a C₁₋₃ alkyl group, and is preferably a hydrogen atom or a methyl group, preferably a hydrogen atom, an ethyl group, or a propyl group, and more preferably a hydrogen atom.

X² represents an oxygen atom, a sulfur atom, or N-R⁷, and is preferably an oxygen atom, a sulfur atom, or NH, and more preferably an oxygen atom.

n represents an integer of 0 to 3, preferably an integer of 1 to 3, and more preferably 1.

R⁵ represents a hydrogen atom, a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent, and from a viewpoint of imparting the hydrophobicity to the structural unit (B), preferably a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent, more preferably a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent, and still more preferably a C₁₋₁₈ alkyl group, a 3- to 8-membered cycloalkyl group, an adamantyl group, or a C₆₋₁₈ aryl group. Meanwhile, a 3- to 8-membered cycloalkyl group optionally having a substituent, an adamantyl group, a C₆₋₁₄ aryl group optionally having a substituent, or a 6- to 10-membered heteroaryl group optionally having a substituent is also preferable.

Here, the substituent is preferably one or more types selected from a halogen atom, an alkyl group having 1 to 6 carbon atoms, an alkenyl group having 2 to 6 carbon atoms, and an alkynyl group having 2 to 6 carbon atoms.

R³ in the structural unit (C) represents a hydrogen atom or a C₁₋₃ alkyl group, and is preferably a hydrogen atom or a methyl group, preferably a hydrogen atom, an ethyl group, or a propyl group, and more preferably a hydrogen atom.

X³ represents an oxygen atom, a sulfur atom, or N-R⁷, and is preferably an oxygen atom, a sulfur atom, or NH, and more preferably an oxygen atom.

R⁶ represents a hydrogen atom, a leaving group, or a linker. The leaving group is a group that can detach when the structural unit (C) binds to the drug (physiologically active substance), and the linker is a group that can be used for crosslinking when the structural unit (C) binds to the drug (physiologically active substance). As the leaving group or linker, a C₁₋₁₈ alkyl group optionally having a substituent, a 3- to 8-membered cycloalkyl group optionally having a substituent, or a C₇₋₁₉ aralkyl group optionally having a substituent is preferable. Here, examples of the substituent include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group. Among these groups, the linker is preferably a group having a functional group such as a hydroxyl group, an amino group, a thiol group, or a carboxyl group as a substituent.

Preferred specific examples of the leaving group of R⁶ include a group represented by the following formula (4):

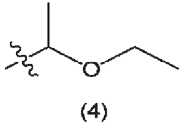

Preferred specific examples of the linker of R⁶ include groups selected from the following formulas (5) to (7).

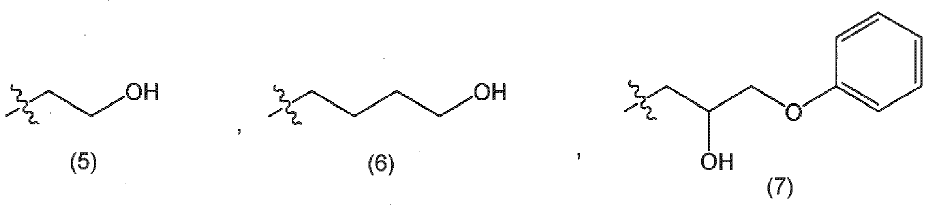

No text.

The copolymer X of the present invention is the copolymer having the structural units represented by the formulas (A), (B) and (C). The copolymer X may be a random copolymer or a block copolymer, and is preferably a random copolymer. As for a composition ratio of each structural unit in one molecule, when (A) is 1 part by mass, preferably (B) is 0.01 to 100 parts by mass and (C) is 0.1 to 100 parts by mass; more preferably (B) is 0.05 to 18 parts by mass and (C) is 0.1 to 20 parts by mass; and particularly preferably (B) is 0.05 to 4 parts by mass and (C) is 0.1 to 16 parts by mass.

A polymerization degree of the copolymer X of the present invention is not particularly limited, and it is preferably 5 000 to 150 000, and more preferably 8 000 to 150 000 as a number average molecular weight.

In the copolymer of the present invention, as described above, the monomer represented by general formula (1) functions as a unit that imparts the hydrophilicity, and the monomer represented by general formula (2) functions as a unit that imparts the hydrophobicity. Further, the monomer represented by general formula (3) functions as a scaffold to which the drug and the copolymer are bonded. Examples of the monomer functioning as the hydrophobic unit represented by general formula (2) include monomers represented by following formulas.

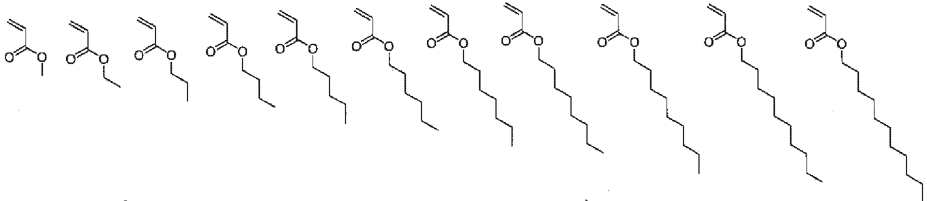

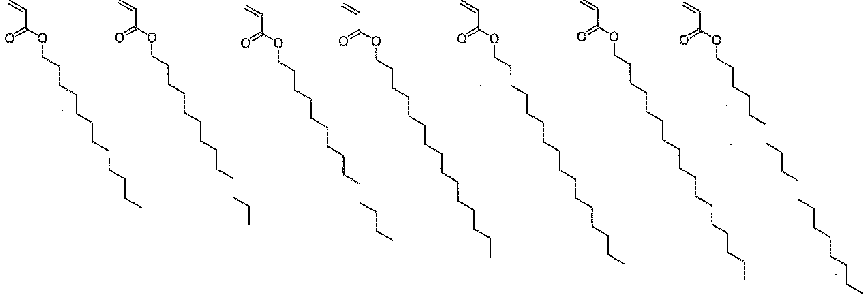

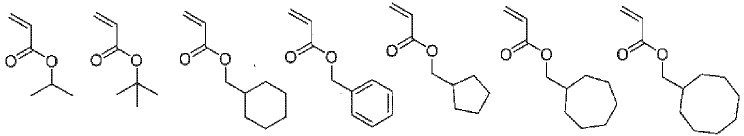

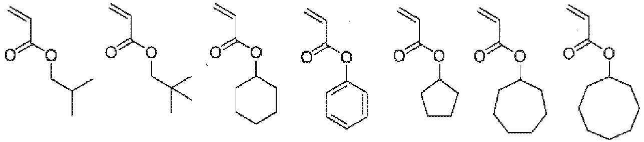

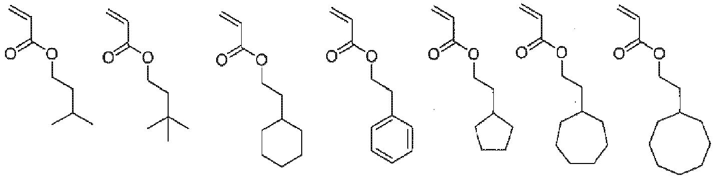

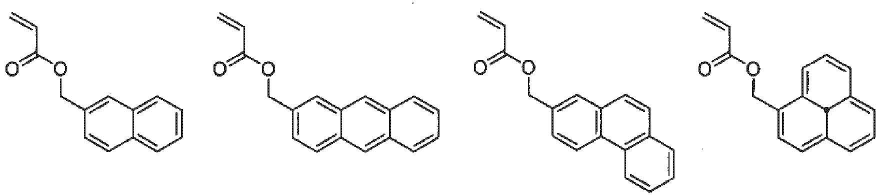

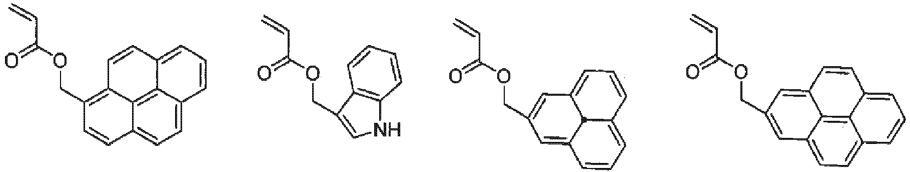

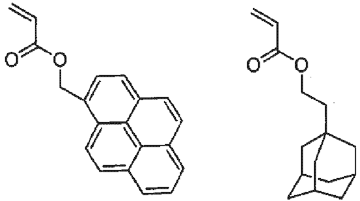

No text.

In general formula (1), R¹ represents a hydrogen atom or a C₁₋₃ alkyl group, and is preferably a hydrogen atom or a methyl group, preferably a hydrogen atom, an ethyl group, or a propyl group, and more preferably a hydrogen atom.

In general formula (2), R² represents a hydrogen atom or a C₁₋₃ alkyl group, and is preferably a hydrogen atom or a methyl group, preferably a hydrogen atom, an ethyl group, or a propyl group, and more preferably a hydrogen atom.

In general formula (3), R³ represents a hydrogen atom or a C₁₋₃ alkyl group, and is preferably a hydrogen atom or a methyl group, preferably a hydrogen atom, an ethyl group, or a propyl group, and more preferably a hydrogen atom.

In general formula (1), R⁴ represents a C₁₋₃ alkyl group, specifically a methyl group, an ethyl group, an n-propyl group or an isopropyl group, preferably a methyl group or an ethyl group, and more preferably a methyl group.

In general formula (1), X¹ represents an oxygen atom, a sulfur atom, or N-R⁷, and is preferably an oxygen atom, a sulfur atom, or NH, and more preferably an oxygen atom.

In general formula (1), m represents an integer of 1 to 100, and is preferably an integer of 3 to 100, and, preferably 3 to 80, more preferably 4 to 60, still more preferably 4 to 40, and still more preferably 4 to 22 from the viewpoint of imparting good hydrophilicity.

In general formula (2), R⁵ represents a hydrogen atom, a C₁₋₁₈ alkyl group, a 3- to 8-membered cycloalkyl group optionally having a substituent, an adamantyl group, a C₆₋₁₈ aryl group optionally having a substituent, or a 5-to 10-membered heteroaryl group optionally having a substituent, and from the viewpoint of imparting hydrophobicity to the structural unit (B), a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent is preferable, a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent is more preferable, and a C₁₋₁₈ alkyl group, a 3- to 8-membered cycloalkyl group, an adamantyl group, or a C₆₋₁₈ aryl group is still more preferable. In addition, a 3- to 8-membered cycloalkyl group optionally having a substituent, an adamantyl group, a C₆₋₁₄ aryl group optionally having a substituent, or a 6-to 10-membered heteroaryl group optionally having a substituent is also preferable. Here, the substituent is preferably one or more types selected from a halogen atom, an alkyl group having 1 to 6 carbon atoms, an alkenyl group having 2 to 6 carbon atoms, and an alkynyl group having 2 to 6 carbon atoms.

In general formula (2), X² represents an oxygen atom, a sulfur atom, or N-R⁷, and is preferably an oxygen atom, a sulfur atom, or NH, and more preferably an oxygen atom.

In general formula (2), n represents an integer of 0 to 3, preferably an integer of 1 to 3, and more preferably 1.

In general formula (3), R⁶ represents a hydrogen atom, a leaving group, or a linker. As the leaving group or linker, a C₁₋₁₈ alkyl group optionally having a substituent, a 3- to 8-membered cycloalkyl group optionally having a substituent, or a C₇₋₁₉ aralkyl group optionally having a substituent is preferable. Here, examples of the substituent include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group. Among these groups, as the linker, a group having a functional group such as a hydroxyl group, an amino group, a thiol group, or a carboxyl group as a substituent is preferable.

Preferred specific examples of the leaving group of R⁶ include a group represented by the following formula (4):

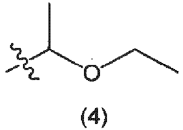

Preferred specific examples of the linker of R⁶ include groups selected from the following formulas (5) to (7).

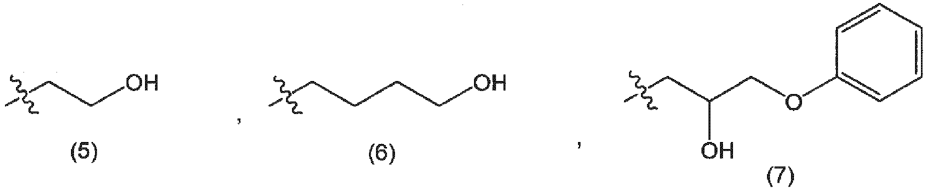

In general formula (3), X³ represents an oxygen atom, a sulfur atom, or N-R⁷, and is preferably an oxygen atom, a sulfur atom, or NH, and more preferably an oxygen atom.

The copolymer X of the present invention is formed by copolymerizing three monomers represented by general formulas (1) to (3). The copolymerization may be random copolymerization or block copolymerization, and those formed by random copolymerization are preferable. As for a blending ratio of the three monomers, it is preferable that 0.01 to 100 parts by mass of monomer (2) and 0.1 to 100 parts by mass of monomer (3) are polymerized, it is more preferable that 0.05 to 18 parts by mass of monomer (2) and 0.1 to 20 parts by mass of monomer (3) are polymerized, and it is particularly preferable that 0.05 to 4 parts by mass of monomer (2) and 0.1 to 16 parts by mass of monomer (3) are polymerized, with respect to 1 part by mass of monomer (1) .

In addition, "solvates" in which various solvents are coordinated are also included in the copolymer X of the present invention. In the present description, examples of the "solvate" include a hydrate and an ethanolate. The solvent may be coordinated to the copolymer X of the present invention in any number.

The copolymer X of the present invention can be produced by various known methods. The production method is not particularly limited, and for example, the copolymer X can be produced according to a basic polymer synthesis method described below. 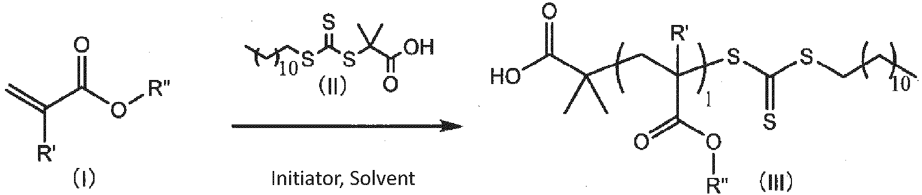 wherein R' represents a hydrogen atom or a C₁₋₃ alkyl group, and R" represents a group represented by R⁴, R⁵ or R⁶.

This reaction shows a step of producing a polymer (III) by reacting a monomer (I) with a chain transfer agent (II) and an initiator. This reaction can be performed in the absence of a solvent or in a solvent such as alcohols such as methanol, ethanol, 1-propanol, and 2-propanol; ethers such as diethyl ether, tetrahydrofuran, and 1,4-dioxane; aromatic hydrocarbons such as benzene, toluene, and xylene; halogenated hydrocarbons such as dichloromethane, chloroform, and 1,2-dichloroethane; N,N-dimethylformamide; N,N-dimethylacetamide; N-methylpyrrolidone; acetonitrile; and ethyl acetate, and it is preferable to use aromatic hydrocarbons such as toluene and xylene as a solvent.

As the chain transfer agent, it is possible to use 2-(dodecylthiocarbonothioylthio)-2-methylpropionic acid (DDMAT), cyanomethyl dodecyltrithiocarbonate (CDTTC), 2-cyano-2-propyldodecyl trithiocarbonate (CPDTTC), 4-cyano-4-[(dodecylsulfanyl-thiocarbonyl)sulfanyl]pentanoic acid (CDSPA), 2-(dodecylthiocarbonothioylthio)-2-methylpropionic acid 3-azido-1-propanol ester (N₃-CTA), and it is preferable to use DDMAT or N₃-CTA, and more preferably N₃-CTA. Where polymerization is carried out by using a chain transfer agent, the copolymer X of the present invention has a structure in which a part or all of a structure of the chain transfer agent is partially bonded. Where the copolymer X includes a structure of a chain transfer agent, the structure may be removed by an appropriate method.

As the initiator, an azo polymerization initiator such as 2,2'-azobis-isobutyronitrile (AIBN), 1,1'-azobis(cyclohexanecarbonitrile) (ACHN), 2,2'-azobis-2-methylbutyronitrile (AMBN), 2,2'-azobis-2,4-dimethylvaleronitrile (ADVN) can be used, and AIBN is preferably used.

The reaction temperature is 0 to 300°C, preferably 0 to 150°C, and more preferably 1 to 100°C, and the reaction time is 1 minute to 48 hours, and preferably 5 minutes to 24 hours. In this reaction, a random copolymerized copolymer X can be produced by carrying out the reaction in the coexistence of monomers (I) having different structures.

For example, if the N₃-CTA having an azide group is used as the chain transfer agent, the copolymer X having an end at which the azide group is present will be obtained, which is advantageous for production of the target-affinity copolymer by a click reaction described later.

The target-affinity molecule bonded to the copolymer X is biotin or the derivative thereof having a specific binding function to avidin/streptavidin or the mutant (modified) form or functional fragment thereof.

Examples of biotin or the derivative thereof include biotin, norbiotin, homobiotin, iminobiotin, oxybiotin, thiobiotin, ethylbiotin, desthiobiotin, diaminobiotin, biotin carbamate, biotin carbonate, trisnorbiotin sulfoxide, bisnorbiotin sulfoxide, norbiotin sulfoxide, biotin sulfoxide, homobiotin sulfoxide, bishomobiotin sulfoxide, trishomobiotin sulfoxide, biotin sulfone, reduced biotin (biotinol), and compounds described in JP S58-154508 A and WO 2014/129446 A. Further, biocytin or a compound having a spacer as described in Avidin-Biotin Technical Handbook from Thermo Fisher can also be used. Moreover, examples of a bis-biotin derivative having high affinity for a streptavidin mutant having reduced affinity for natural biotin include compounds described in WO 2015/125820 A, WO 2018/151301 A, WO 2019/189867 A, WO 2019/230905 A, and WO 2021/210573 A, and are not limited thereto.

Here, biotin or the derivative thereof is preferably a molecule having a biotin residue represented by the following formula (a): 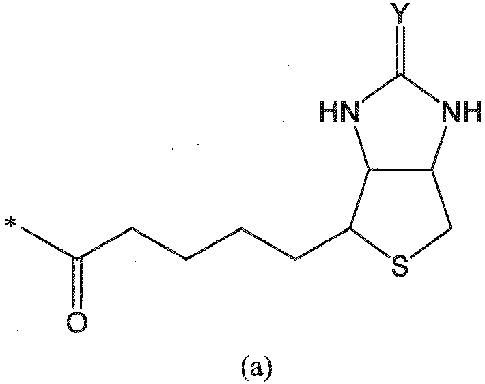 wherein, Y represents O or NH.

Examples of biotin or the derivative thereof can preferably include compounds represented by the following formulas (8) to (11).

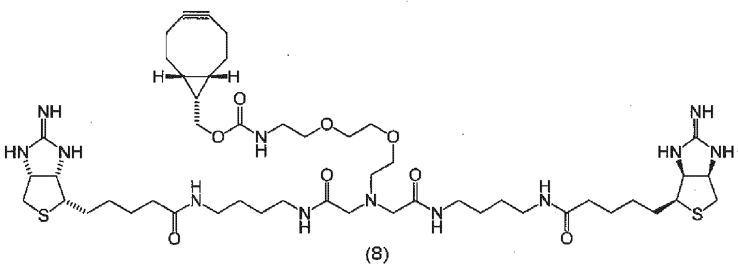

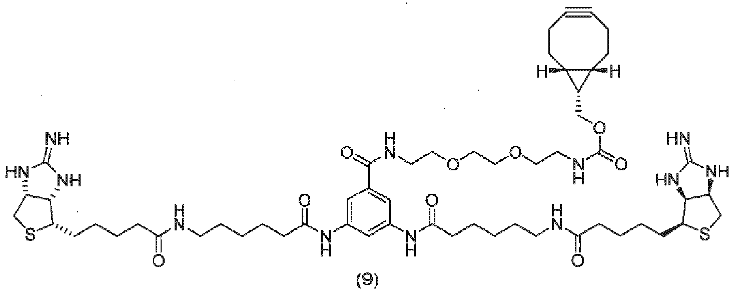

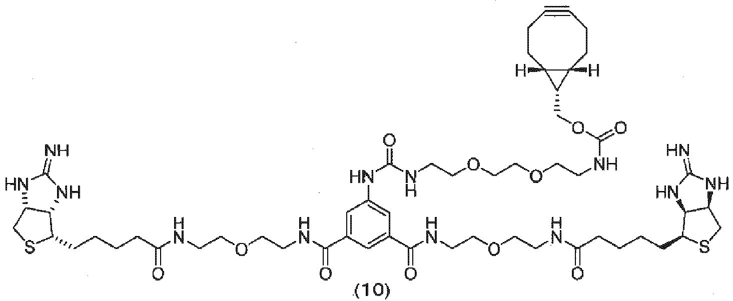

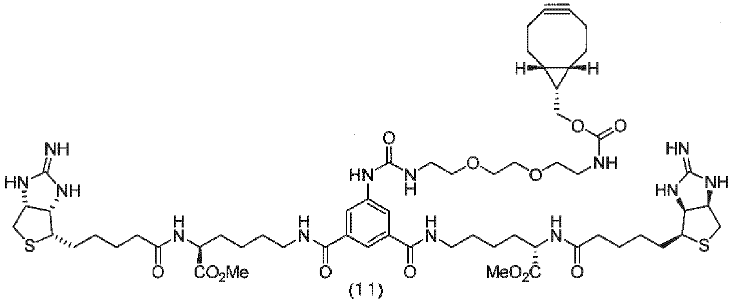

Examples of biotin or the derivative thereof is more preferably the compound represented by the following formula (8).

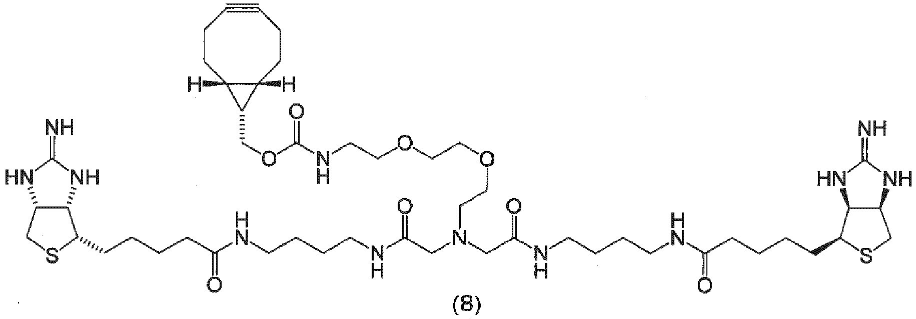

Where geometric isomers or optical isomers are present in the compounds of the present invention, mixtures or separations of those isomers are also included in the scope of the present invention. Separation of the isomers can be performed by a conventional method.

The target-affinity copolymer of the present invention can be produced by various known methods. The production method is not particularly limited, and for example, the target-affinity copolymer can be produced according to a synthesis method through the click reaction described below.

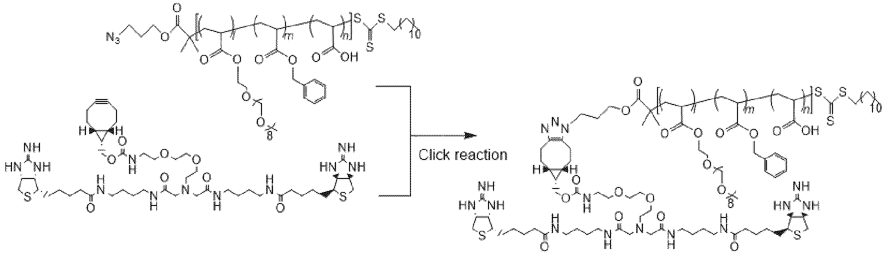

The "click chemistry" is classified to reactions similar to natural biochemical reactions, having the following attributes, and has the following characteristics. The click chemistry is a very efficient reaction that proceeds rapidly to obtain high yields, and is a very selective reaction that produces no (or little) by-products and allows polyfunctional groups. Moreover, the click chemistry proceeds under mild reaction conditions such as a low temperature (or ambient temperature) or in an aqueous solution. This reaction can be performed in water or in a solvent such as an alcohol such as methanol, ethanol, 1-propanol, or 2-propanol; an ether such as diethyl ether, tetrahydrofuran, or 1,4-dioxane; an aromatic hydrocarbon such as benzene, toluene, or xylene; a halogenated hydrocarbon such as dichloromethane, chloroform, or 1,2-dichloroethane; N,N-dimethylformamide; N,N-dimethylacetamide; N-methylpyrrolidone; acetonitrile; or ethyl acetate. It is preferable to use water or N,N-dimethylformamide as the solvent. In some aspects, the cyclooctyne is dibenzocyclooctyne (DBCO), difluorobenzocyclooctyne (DIFBO), biarylazacyclooctynone (BARAC), dibenzocyclooctyne (DIBO), difluorinated cyclooctyne (DIFO), monofluorinated cyclooctyne (MOFO), dimethoxyazacyclooctyne (DIMAC), or aryl-less octyne (ALO), with dibenzocyclooctyne (DBCO) being preferably used. In some aspects, the alkyne is aliphatic alkyne and the reacting step is performed in the presence of a copper (I) catalyst. In some aspects, the alkyne is cyclooctyne and the reacting step is performed under copper-free conditions. The reaction temperature is 0 to 300°C, preferably 0 to 150°C, and more preferably 1 to 100°C, and the reaction time is 1 minute to 48 hours, and preferably 5 minutes to 24 hours.

Reaction of the reaction formula is Huisgen cycloaddition which is a kind of click reaction, and is a 1,3-dipolar cycloaddition reaction in which 1,2,3-triazole is formed from the azide and the alkyne.

The produced polymer X and target-affinity copolymer of the present invention can be purified by a polymer isolation and purification method generally known in the field of polymer chemistry. Specific examples thereof include treatment operations such as extraction, recrystallization, salting out using, for example, ammonium sulfate or sodium sulfate, centrifugation, dialysis, ultrafiltration, adsorption chromatography, ion exchange chromatography, hydrophobic chromatography, normal phase chromatography, reverse phase chromatography, gel filtration, gel permeation chromatography, affinity chromatography, electrophoresis, countercurrent distribution, and combinations thereof.

The copolymer X and the target-affinity copolymer of the present invention can be used as a carrier for transporting of various physiologically active substances (drugs). For example, a pharmaceutical composition in which a therapeutic agent for tumor is carried on (encapsulated in) the copolymer X or the target-affinity copolymer of the present invention can be used as a prophylactic and/or therapeutic agent for various cancer diseases such as a colon cancer, a duodenal cancer, a gastric cancer, a pancreatic cancer, a liver cancer, a lung cancer, a uterine cancer, and an ovarian cancer, because the pharmaceutical composition suppresses growth of tumors, as confirmed in test examples described later. Further, with a high tumor accumulation ability, the pharmaceutical composition can be used as a diagnostic agent or a contrast agent for tumor.

When the copolymer and the target-affinity copolymer of the present invention are used as a drug transport carrier, a dose thereof and the number of administration times thereof may be appropriately selected in consideration of, for example, a dosage form, an age and body weight of a patient, and nature or severity of a symptom to be treated, and the dose and the number of administration times should not be limited. However, where the polymer containing the drug is intravenously injected by an injection, for example, for an adult (60 kg), a single dose is preferably administered in an amount of 0.12 mg to 12 000 000 mg, more preferably 1.2 mg to 1 200 000 mg, and particularly preferably 12 to 120 000 mg.

The pharmaceutical composition of the present invention can be produced by mixing the drug with the copolymer X or the target-affinity copolymer of the present invention. Preferably, the copolymer or the target-affinity copolymer of the present invention may be mixed with the drug to produce the single chain nanoparticle, or the single chain nanoparticle of the copolymer X or the target-affinity copolymer may be produced and then mixed with the drug. The single chain nanoparticle can be produced by a known method.

In the pharmaceutical composition of the present invention, the drug may be carried on the copolymer X or the target-affinity copolymer by the action such as electrostatic interaction, hydrogen bond, hydrophobic interaction, or covalent bond.

The drug may be any drug used for various cancer diseases, preferably an anticancer agent that acts on cancer cells to suppress proliferation of the cancer cells, and examples thereof include an antimetabolite, an alkylating agent, an anthracycline, an antibiotic, a mitotic inhibitor, a topoisomerase inhibitor, a proteasome inhibitor, and an anti-hormone agent.

Examples of the antimetabolite include azathioprine, 6-mercaptopurine, 6-thioguanine, fludarabine, pentostatin, cladribine, 5-fluorouracil (5FU), floxuridine (FUDR), cytosine arabinoside (cytarabine), methotrexate, trimethoprim, pyrimethamine, and pemetrexed. Examples of the alkylating agent include cyclophosphamide, mechlorethamine, uramustine, melphalan, chlorambucil, thiotepa/chlorambucil, ifosfamide, carmustine, lomustine, streptozocin, busulfan, dibromomannitol, cisplatin, carboplatin, nedaplatin, oxaliplatin, miriplatin, satraplatin, triplatin tetranitrate, procarbazine, altretamine, dacarbazine, mitozolomide, trabectedin, and temozolomide. Examples of the anthracycline include daunorubicin, doxorubicin, epirubicin, idarubicin, valrubicin, aclarubicin, amrubicin, and pirarubicin. Examples of the antibiotic include dactinomycin, bleomycin, mithramycin, anthramycin, streptozotocin, gramicidin D, staurosporine, mitomycins (for example, mitomycin C), duocarmycins (for example, CC-1065), and calicheamicins. Examples of the mitotic inhibitor include maytansinoids (for example, DM0, mertansine (also known as DM1), DM2, DM3, DM4, or emtansine), auristatins (for example, auristatin E, auristatin phenylalanine phenylenediamine (AFP), monomethyl auristatin E, monomethyl auristatin D, or monomethyl auristatin F), dolastatins, cryptophycins, vinca alkaloid (for example, vincristine, vinblastine, vindesine, or vinorelbine), taxanes (for example, paclitaxel or docetaxel), and colchicines. Examples of the topoisomerase inhibitor include irinotecan, topotecan, nogitecan, amsacrine, etoposide, teniposide, mizantrone, mitoxantrone, SN-38, exatecan, and deruxtecan. Examples of the proteasome inhibitor can include a peptidyl boronic acid, carfilzomib, and bortezomib. Examples of the anti-hormone agent can include fulvestrant, tamoxifen, and toremifene. Where these drugs are used to prepare the pharmaceutical composition of the present invention, one or more of the drugs can also be used in combination, and the drugs may be carried on the copolymer X or the target-affinity copolymer as a free form.

A route of administration of the pharmaceutical composition of the present invention is desirably the most effective one for treatment, and the pharmaceutical composition can be administered by a parenteral administration preparation such as an oral administration preparation, an injection, or a transdermal administration preparation. For example, parenteral administration such as intraarterial injection, intravenous injection, subcutaneous injection, intramuscular injection, or intraperitoneal injection is preferable, and intraarterial injection and intravenous injection are more preferable. The number of doses should not be limited, and examples thereof include one to several doses per week average.

Various preparations suitable for the route of administration can be produced by a conventional method by appropriately selecting preparation additives such as excipients, extenders, binders, wetting agents, disintegrants, lubricants, surfactants, dispersants, buffers, preservatives, solubilizing agents, antiseptics, flavoring agents, soothing agents, stabilizers, and isotonizing agents that are conventionally used in formulation.

The preparation additives that can be contained in the various preparations described above are not particularly limited as long as the preparation additives are pharmaceutically acceptable. Examples of such preparation additives include purified water, water for injection, distilled water for injection, pharmaceutically acceptable organic solvents, collagen, polyvinyl alcohol, polyvinylpyrrolidone, carboxyvinyl polymer, sodium alginate, water-soluble dextran, sodium carboxymethyl starch, pectin, xanthan gum, gum arabic, casein, gelatin, agar, glycerin, propylene glycol, polyethylene glycol, petrolatum, paraffin, stearyl alcohol, stearic acid, human serum albumin, mannitol, sorbitol, and lactose. Additives to be used are appropriately selected according to various preparations, and can be used alone or in combination.

The injection can also be prepared as a non-aqueous diluent (for example, polyethylene glycol, vegetable oils such as olive oil, and alcohols such as ethanol), a suspension, or an emulsion. Sterilization of the injection can be performed by filtration sterilization using a filter, and blending of, for example, a microbicide. In addition, the injection can be produced in a form of preparation before use. That is, the injection can be formed into a sterile solid composition by, for example, lyophilization, and can be dissolved in water for injection, distilled water for injection, or another solvent before use.

In a therapeutic method in which the target-affinity copolymer of the present invention is used, first, the fusion of the molecular probe such as the cancer antigen-specific antibody molecule and avidin/streptavidin or the mutant (modified) form or functional fragment thereof is prepared, and administered to the patient to accumulate the fusion on the cancer cell. Next, a process is performed to administer a conjugate of the target-affinity copolymer of the present invention and an anticancer agent to the patient, thereby accurately accumulating the drug on the cancer cell.

It is also possible to prepare a complex in which a conjugate of the molecular probe such as the cancer antigen-specific antibody molecule and the avidin/streptavidin or the mutant (modified) form or functional fragment thereof is bonded to a conjugate of the target-affinity copolymer of the present invention and the anticancer agent in advance and administer the complex to

the patient.

### Examples

Hereinafter, the present invention will be described more specifically with reference to examples. These examples are provided for purpose of exemplification and are not intended to limit embodiments of the invention.

### [Example 1] Production of poly[(benzyl acrylate)-co-(poly(ethylene glycol)methyl ether acrylate)-co-(1-ethoxyethyl acrylate)]

### (1) Synthesis of 1-ethoxyethyl acrylate (EEA)

Ethyl vinyl ether (28.725 mL) was weighed under an argon atmosphere, and thereto was added a phosphoric acid (50 mg) under ice cooling. Thereto was added acrylic acid (17.15 mL), and the mixture was stirred at room temperature for 48 hours. Further thereto was added hydrotalcite (3 g), and the mixture was stirred for 2 hours, and the reaction was stopped. After celite filtration, unreacted ethyl vinyl ether was removed by evaporation. Thereto was added phenothiazine (up to 500 ppm) as a polymerization inhibitor, and the mixture was purified by distillation under a reduced pressure together with calcium hydride (distillation temperature of 28 to 32°C). The obtained 1-ethoxyethyl acrylate was dispensed into a glass vial and stored at -30°C.

¹³C NMR (400MHz, CDCl₃), δ, ppm: 15.29 (-OCH₂CH₃), 21.16 (-COOCH(CH₃)), 64.98 (-OCH₂-), 96.73 (-COOCH(CH₃)), 128.84 (CH₂CH-), 131.43 (CH₂CH-), 166.00 (-COO).

### (2) Synthesis of poly[(benzyl acrylate)-co-(poly(ethylene glycol) methyl ether acrylate)-co-(1-ethoxyethyl acrylate)]

100 mg of 2-(dodecylthiocarbonothioylthio)-2-methylpropionic acid (DDMAT) was weighed and dissolved in 17.3 mL of toluene to prepare a DDMAT/toluene stock solution (5.78 mg/mL as a DDMAT concentration). Similarly, 22 mg of 2,2'-azobis(2-methylpropionitrile) (AIBN) was weighed and dissolved in 17.3 mL of toluene to prepare an AIBN/toluene stock solution (AIBN concentration: 1.27 mg/mL). Separately, 1.296 g of poly(ethylene glycol) methyl ether acrylate (mPEGA, the average value (n) of the numbers of repetitions of ethylene glycol is 9), 0.394 g of benzyl acrylate (BnA), 0.039 g of 1-ethoxyethyl acrylate, 1.73 mL of a DDMAT/toluene stock solution, and 1.73 mL of an AIBN/toluene stock solution were added, and polymerization was performed in an oil bath at 70°C. After a lapse of 90 minutes, the polymerization was stopped, and then the reaction solution was subjected to a reprecipitation method or dialyzed against methanol to recover the copolymer. Since the obtained copolymer was basically a viscous body, in the reprecipitation method, an operation of dropping the reaction solution into a centrifuge tube to which a poor solvent (hexane/ethyl acetate = 7/3 [v/v]) was added and recovering the solution by centrifugation (2 000 × g, 5 min) was repeated 3 times, and finally vacuum drying was performed to obtain 1.223 g of poly[(benzyl acrylate)-co-(poly(ethylene glycol) methyl ether acrylate)-co-(1-ethoxyethyl acrylate)].

As a result of analyzing the polymerization degree of each monomer and the number average molecular weight (M_{n,NMR}) from a ¹H-NMR spectrum of the obtained copolymer measured using NMR, the polymerization degree of mPEGA (n = 9) was 102, the polymerization degree of BnA was 94, the polymerization degree of EEA was 9, and M_{n,NMR} was 65 900. The molecular weight dispersion (M_{w}/Mₙ) of the obtained copolymer was measured using GPC, and as a result, it was found to be 1.53.

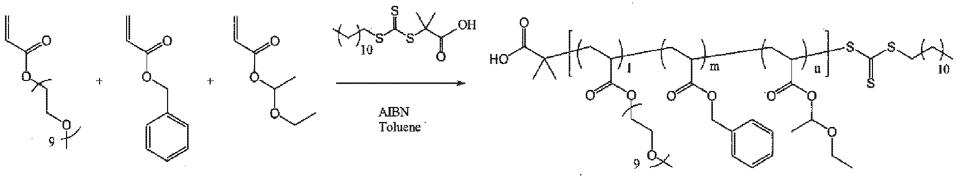

### [Measurement apparatus and conditions]

### (1) ¹H-NMR measurement

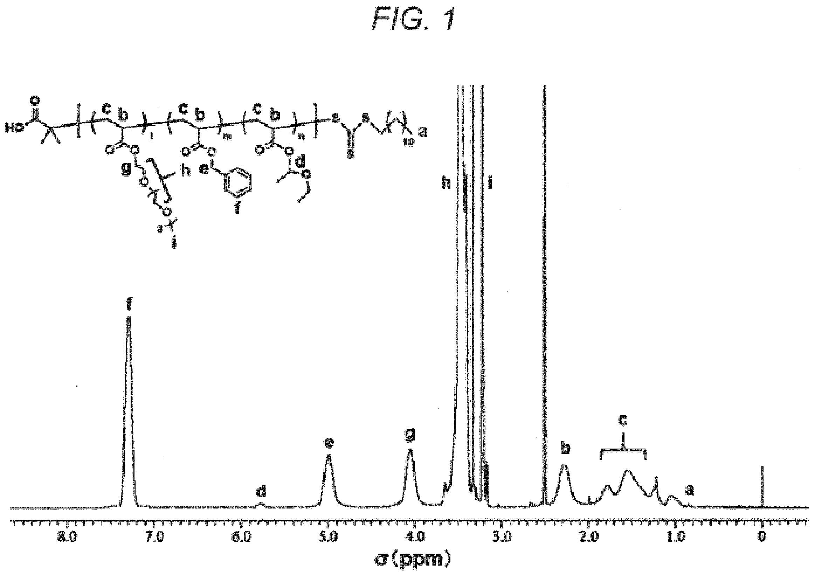

Apparatus: JNM-ECX 400 (400 MHz)/JEOL Ltd.
Solvent: Dimethyl sulfoxide-d₆ containing 0.03% tetramethylsilane/KANTO CHEMICAL CO., INC.
Sample concentration: 20 mg/mL
Measurement temperature: 25°C
Number of integration times: 256 times
Result: Fig. 1

### (2) GPC measurement

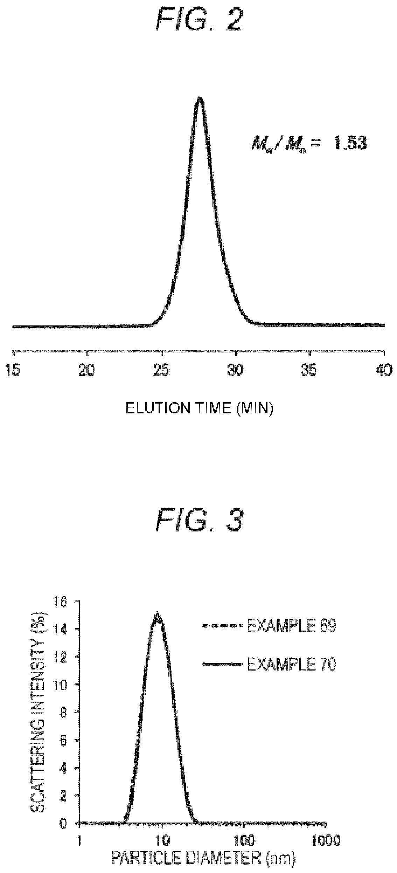

Apparatus: HPLC-Prominence system/SHIMADZU CORPORATION
Detector: RID-10A Refractive index detector/SHIMADZU CORPORATION
Column: TSKgel α-2500 column/Tosoh Corporation
(Column size: 7.8 mm × 300 mm, particle size: 7 µm,
exclusion limit molecular weight: 5 × 10³)
TSKgel α-4000 column/Tosoh Corporation
(Column size: 7.8 mm × 300 mm, particle size: 10 µm,
exclusion limit molecular weight: 4 × 10⁵)
TSKgel guard column/Tosoh Corporation
Mobile phase: N,N-dimethyformamide (DMF) containing 10 mmol/L lithium bromide
Temperature: 40°C
Flow rate: 0.5 mL/min
Sample concentration: 6 mg/mL
Standard substance: Poly(methyl methacrylate) standard ReadyCal set, Mₚ 800 - 2 200 000 Da/SIGMA
Result: Fig. 2

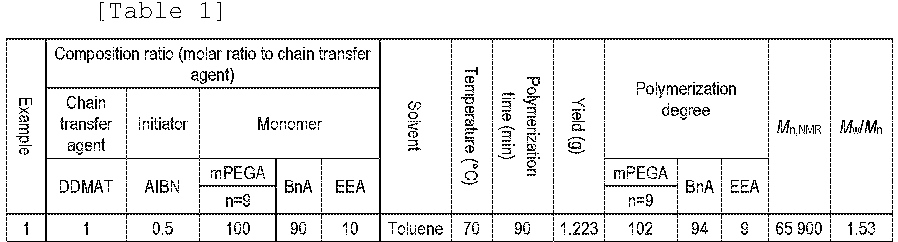

### [Examples 2 to 68]

Polymers having different composition ratios and average molecular weights shown in the following table were produced by appropriately changing the type, charged amount, reaction temperature, and polymerization time of the monomers (mPEGA, BnA, and EEA) used in Example 1, and using the same method as in Example 1.

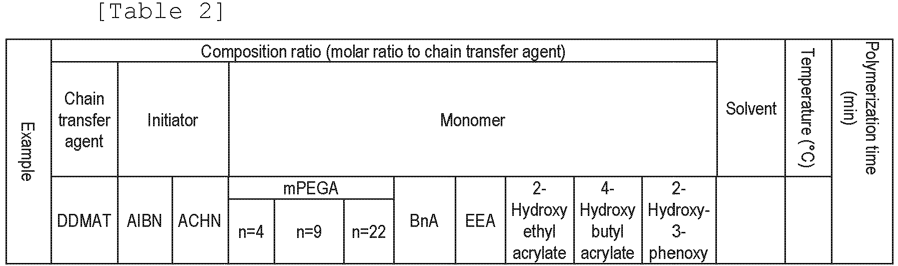

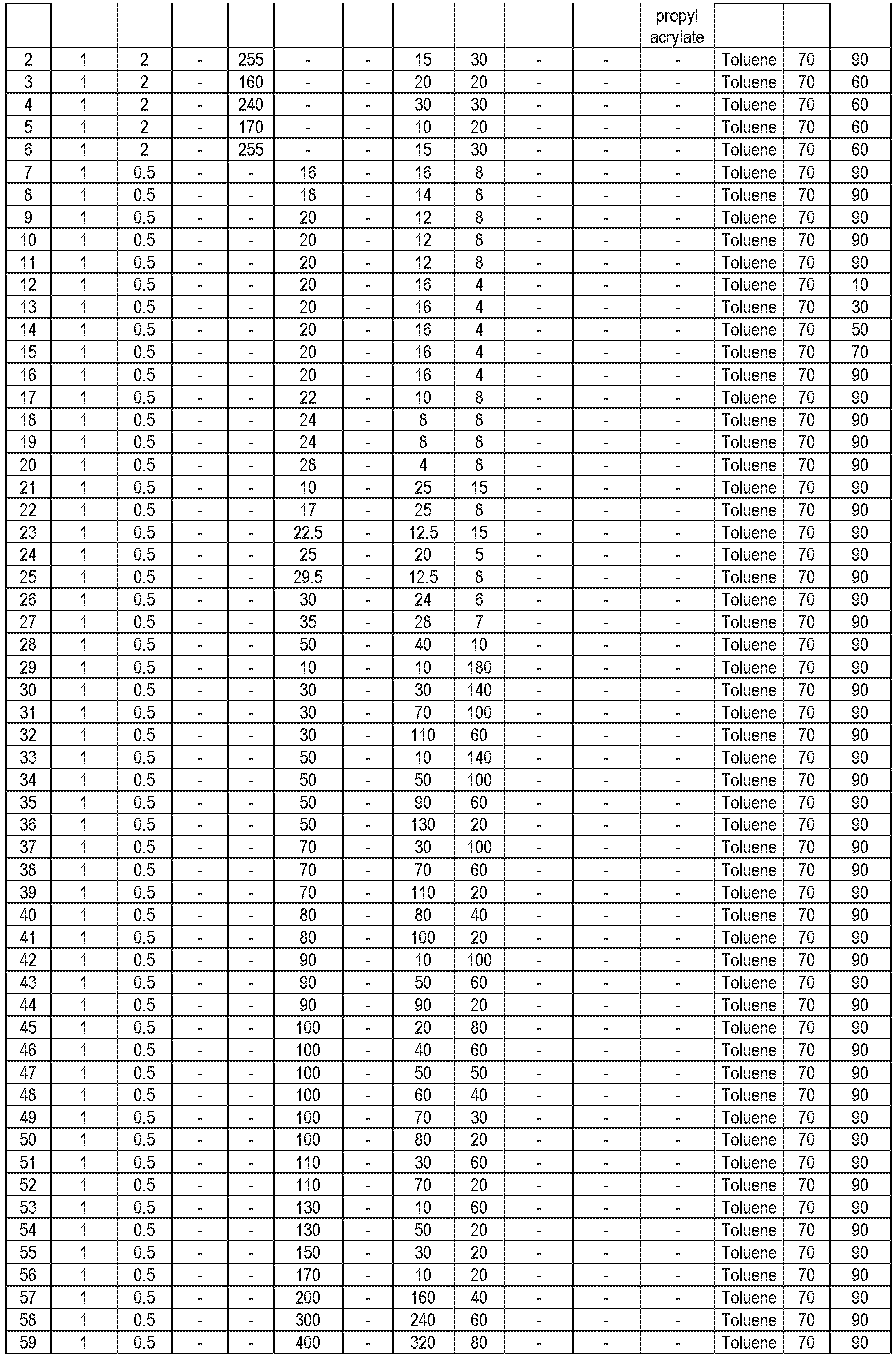

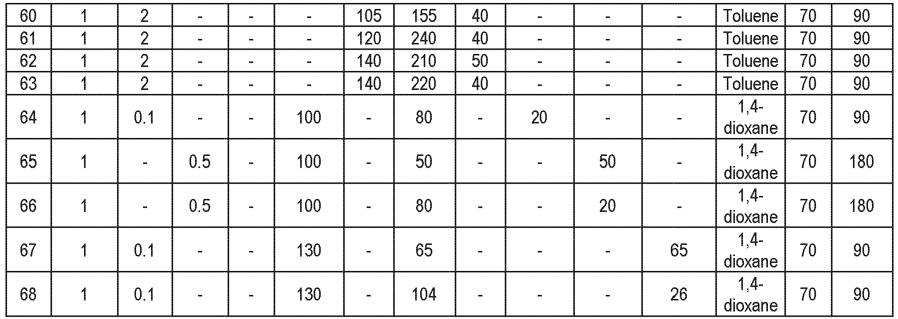

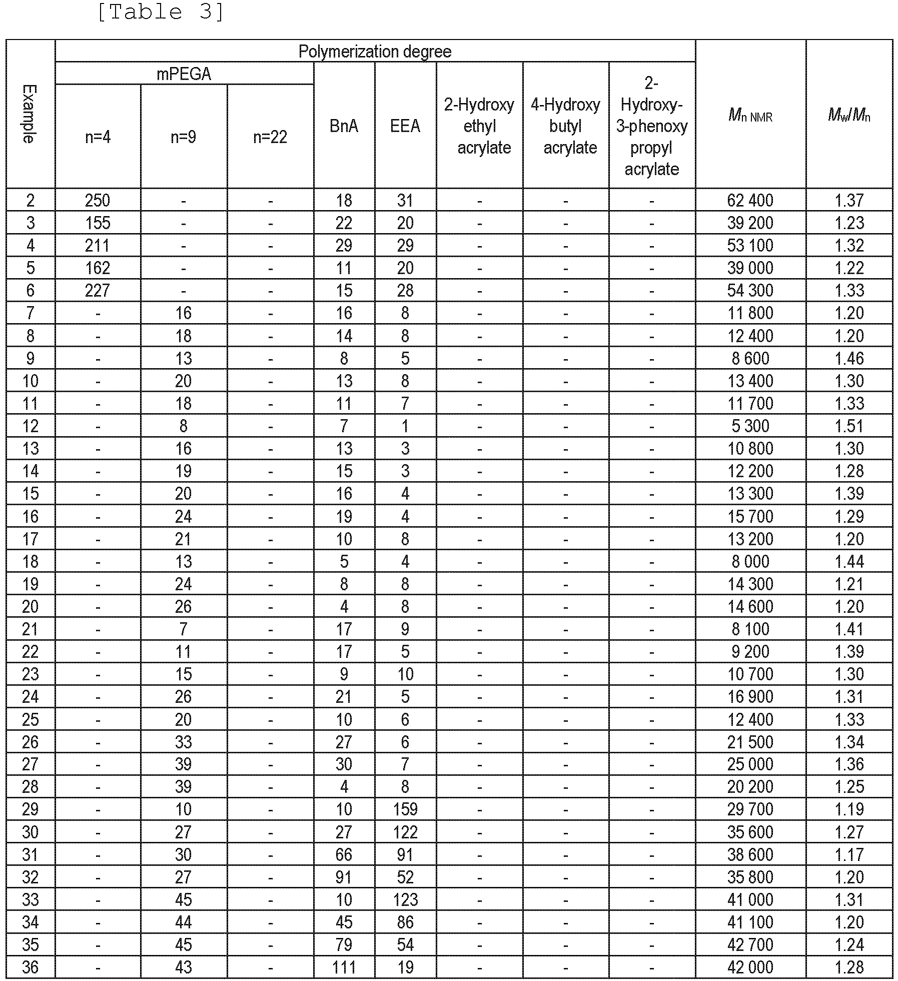

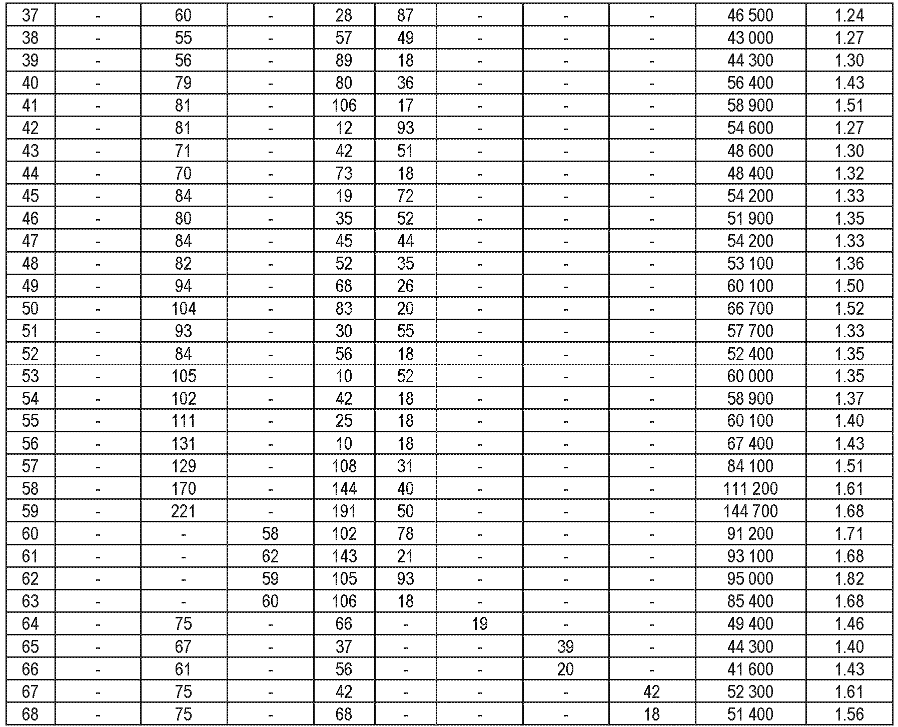

### [Example 69]

### Production of poly[(benzyl acrylate)-co-(poly(ethylene glycol)methyl ether acrylate)-co-(acrylic acid)]

By treating poly[(benzyl acrylate)-co-(poly(ethylene glycol)methyl ether acrylate)-co-(1-ethoxyethyl acrylate)] obtained in Example 1 with 0.5N HCl at room temperature, an ethoxyethyl group was eliminated to obtain a terpolymer having a carboxyl group (1.176 g). A Z-average particle diameter and a polydispersity index of the obtained terpolymer in water were measured by dynamic light scattering (DLS), and as a result, the Z-average particle diameter was 8.5 nm (polydispersity index: 0.14).

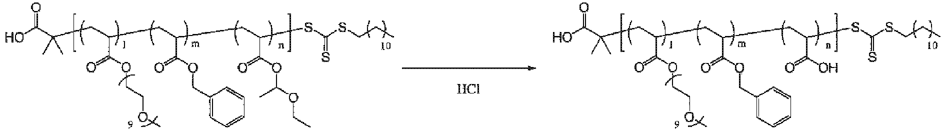

### [Measurement apparatuses and conditions]

### (1) DLS measurement

Apparatus: Zetasizer NanoZS/Malvern Instruments Ltd.
Measurement temperature: 25°C
Sample concentration: 10 mg/mL
Results: Fig. 3

### [Example 70]

### Method for producing (1,2-diaminocyclohexane)platinum(II)-encapsulating SCNP

65.28 mg of a Cl(H₂O) form of (1,2-diaminocyclohexane)platinum(II) (hereinafter, abbreviated as DACHPt) (DACHPt·Cl·H₂O) was dissolved in 20 mL of purified water and stirred at 70°C for 2 hours. To 5 mL of this solution, 287.4 mg of the terpolymer obtained in Example 69 was added, and the mixture was stirred at 50°C overnight. After completion of stirring, the reaction solution was dialyzed and purified using purified water as an external solution to obtain 5 mL of a DACHPt-encapsulating SCNP. The Pt content of the DACHPt-encapsulating SCNP obtained after purification was measured by inductively coupled plasma-atomic emission spectrometry (ICP-AES) and found to be 720 ug/mL (1.14 mg/mL as DACHPt). Separately, 200 µL of the DACHPt-encapsulating SCNP was lyophilized and the solid content concentration was calculated, then the ratio to the Pt content was taken, and the Pt binding amount per polymer was calculated. As a result, the Pt binding amount was 3.4 mol/mol. In addition, the Z-average particle diameter and the polydispersity index of the obtained DACHPt-encapsulating SCNP were measured by dynamic light scattering (DLS), and as a result, the Z-average particle diameter was found to be 8.7 nm (polydispersity index: 0.14). The particle diameter of the SCNP before and after encapsulation of DACHPt is shown in Fig. 3. The particle diameter of the SCNP hardly varied before and after encapsulation of DACHPt. The results are summarized in the following table.

### [Measurement apparatuses and conditions]

### (1) ICP-AES measurement

Apparatus: Sequential high-frequency plasma light-emitting apparatus ICPE-9000/SHIMADZU CORPORATION
Pretreatment apparatus: microwave sample pretreatment apparatus ETHOS EASY/Milestone General
Measurement wavelength: 214 nm
Standard solution: Platinum standard solution (Pt1000) for ICP analysis/FUJIFILM Wako Pure Chemical Corporation

### 2) DLS measurement

Apparatus: Zetasizer NanoZS/Malvern Instruments Ltd.
Measurement temperature: 25°C
Sample concentration: 10 mg/mL
Results: Fig. 3

**[Table 4]**

| Example | Yield (mL) | Pt contect (µg/mL) | Pt contect per polymer (mol/mol) | Z-average particle dimeter (nm) | Polydispersity Index |
|---|---|---|---|---|---|
| 70 | 5 | 720 | 3.4 | 8.7 | 0.14 |

### [Example 71]

### Production of N₃-poly[(benzyl acrylate)-co-(poly(ethylene glycol)methyl ether acrylate)-co-(1-ethoxyethyl acrylate)]

Synthesis was performed by using N₃-CTA in place of the chain transfer agent DDMAT used in Example 1. In toluene (17.3 mL), 2-(dodecylthiocarbonothioylthio)-2-methylpropionic acid 3-azido-1-propanol ester (N₃-CTA) (100 mg) was dissolved after being weighed to obtain an N₃-CTA/toluene stock solution (N₃-CTA concentration: 5.78 mg/mL). Similarly, 2,2'-azobis(2-methylpropionitrile) (AIBN) (10 mg) was weighed and dissolved in toluene (7.87 mL) to obtain an AIBN/toluene stock solution (AIBN concentration: 1.27 mg/mL). Separately, poly(ethylene glycol)methyl ether acrylate (mPEGA, average value (n) of the numbers of repetition times of ethylene glycol is 9) (2.592 g), benzyl acrylate (BnA) (0.684 g), 1-ethoxyethyl acrylate (0.172 g), the N₃-CTA/toluene stock solution (4.15 mL), and the AIBN/toluene stock solution (3.46 mL) were added, and polymerization was performed in an oil bath at 70°C. After a lapse of 90 minutes, the polymerization was stopped, and then a copolymer was recovered by subjecting the reaction solution to reprecipitation or dialysis against methanol. Since the obtained copolymer was basically a viscous form, for the reprecipitation, an operation of dropping the reaction solution into a centrifuge tube to which the poor solvent (hexane/ethyl acetate = 7/3 [v/v]) was added and recovering the solution by centrifugation (2 000 × g, 5 min) was repeated three times, and finally the vacuum drying was performed to obtain N₃-poly[(benzyl acrylate)-co-(poly(ethylene glycol)methyl ether acrylate)-co-(1-ethoxyethyl acrylate)] (2.643 g).

As a result of analyzing a polymerization degree of each of monomers and a number average molecular weight (M_{n,NMR}) of the obtained copolymer from a ¹H-NMR spectrum measured by using NMR, the polymerization degree of mPEGA (n = 9) was 70, the polymerization degree of BnA was 56, the polymerization degree of EEA was 15, and M_{n,NMR} was 44 000. Moreover, a molecular weight dispersion (M_{w}/Mₙ) of the obtained copolymer was measured by using the GPC and a result thereof was 1.32.

### [Example 72]

### Production of N₃-poly[(benzyl acrylate)-co-(poly(ethylene glycol)methyl ether acrylate)-co-(acrylic acid)]

By treating N₃-poly[(benzyl acrylate)-co-(poly(ethylene glycol)methyl ether acrylate)-co-(1-ethoxyethyl acrylate)] obtained in Example 71 with 0.5N HCl at room temperature, an ethoxyethyl group was eliminated to obtain a terpolymer having a carboxyl group (2.455 g). A Z-average particle diameter and a polydispersity index of the obtained terpolymer in water were measured by dynamic light scattering (DLS), and a result of the Z-average particle diameter was 7.5 nm (polydispersity index: 0.18).

### [Example 73]

### Production of biotin-bonded SCNP

To DMF (5 mL) in a glass vial, were added N₃-poly[(benzyl acrylate)-co-(poly(ethylene glycol) methyl ether acrylate)-co-(acrylic acid)] (275 mg) and ((1R,8S,9S)-bicyclo[6.1.0]non-4-yn-9-yl)methyl(22-((3aS,4S,6aR)-2-iminohexahydro-1H-thieno[3,4-d]imidazol-4-yl)-9-(2-((4-(5-((3aS,4S,6aR)-2-iminohexahydro-1H-thieno[3,4-d]imidazol-4-yl)pentanamido)butyl)amino)-2-oxoethyl)-11,18-dioxo-3,6-dioxa-9,12,17-triazadocosyl)carbamate (19.5 mg) and the mixture was stirred overnight at room temperature. The reaction solution was dialyzed against methanol, the solvent was distilled off, and then the biotin-bonded SCNP (265.7 mg) was recovered by the vacuum drying. A Z-average particle diameter and a polydispersity index of the biotin-bonded SCNP were measured by dynamic light scattering (DLS), and a result of the Z-average particle diameter was 8.6 nm (polydispersity index: 0.17).

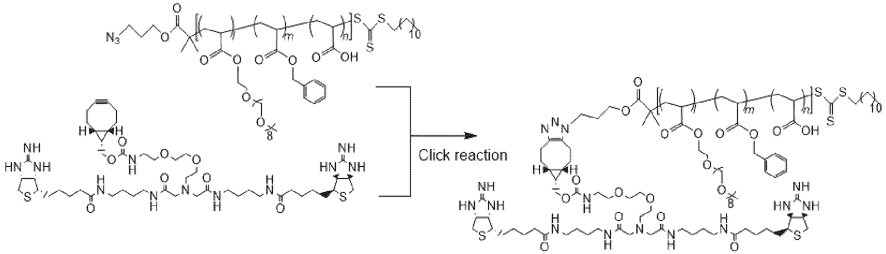

### [Measurement apparatuses and conditions]

### (1) ¹H-NMR measurement

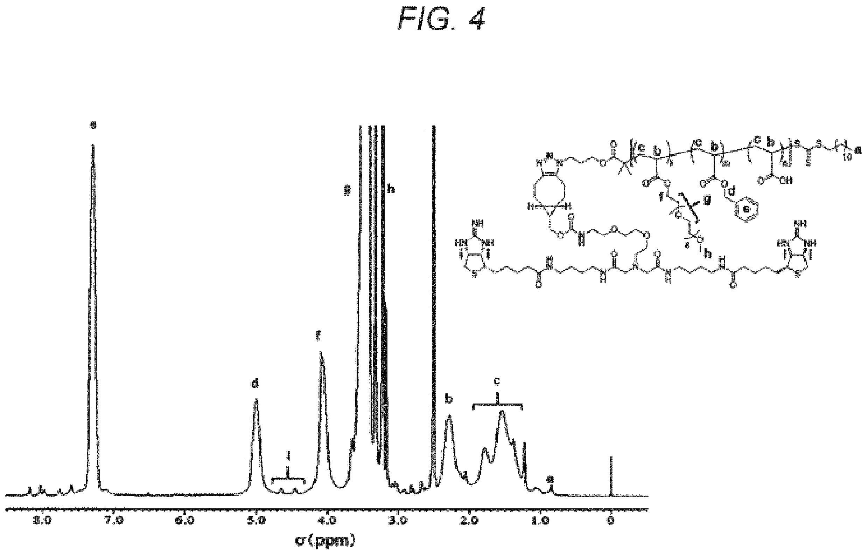

Apparatus: JNM-ECX400 (400 MHz)/JEOL Ltd.
Solvent: dimethyl sulfoxide-d₆ containing 0.03% tetramethylsilane/KANTO CHEMICAL CO., INC.
Sample concentration: 20 mg/mL
Measurement temperature: 25°C
Number of integration times: 6 000 times
Results: Fig. 4

### (2) GPC measurement

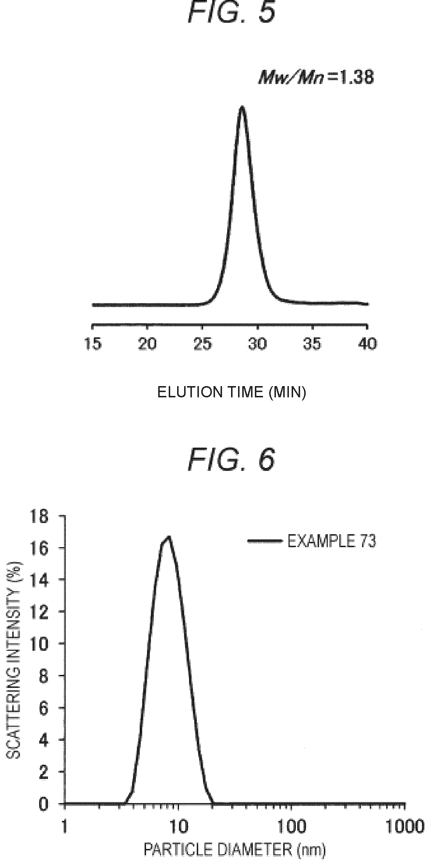

Apparatus: HPLC-Prominence system/SHIMADZU CORPORATION
Detector: RID-10A Refractive index detector/SHIMADZU CORPORATION
Column: TSKgel α-2500 column/Tosoh Corporation
(Column size: 7.8 mm × 300 mm, particle size: 7 µm,
exclusion limit molecular weight: 5 × 10³)
TSKgel α-4000 column/Tosoh Corporation
(Column size: 7.8 mm × 300 mm, particle size: 10 µm,
exclusion limit molecular weight: 4 × 10⁵)
TSKgel guard column/Tosoh Corporation
Mobile phase: N,N-dimethyformamide (DMF) containing 10 mmol/L lithium bromide
Temperature: 40°C
Flow rate: 0.5 mL/min
Sample concentration: 6 mg/mL
Standard substance: Poly(methyl methacrylate) standard ReadyCal set, Mₚ 800 - 2 200 000 Da/SIGMA
Result: Fig. 5

### (3) DLS measurement

Apparatus: Zetasizer NanoZS/Malvern Instruments Ltd.
Measurement temperature: 25°C
Sample concentration: 10 mg/mL
Results: Fig. 6

### [Example 74]

### Synthesis of ((1R,8S,9s)-bicyclo[6.1.0]non-4-yn-9-yl)methyl(2-(2-(2-(3,5-bis(6-(5-((3aS,4S,6aR)-2-iminohexahydro-1H-thieno[3,4-d]imidazol-4-yl)pentanamido)hexanamido)benzamido)ethoxy)ethoxy)ethyl)car bamate di(2,2,2-trifluoroacetate)

To (3aS,3a'S,4S,4'S,6aR,6a'R)-4,4'-{5,5'-[6,6'-(5-(2-(2-(2-aminoethoxy)ethoxy)ethylcarbamoyl)-1,3-phenylene)bis(azanediyl)bis(6-oxohexane-6,1-diyl)]bis(azanediyl)bis(5-oxopentane-5,1-diyl) }bis(tetrahydro-1H-thieno[3,4-d]imidazol-2(3H)-iminium)tri(2,2,2-trifluoroacetate) (415 mg), DMF (15 mL), triethylamine (445 µL), and (1R,8S,9S)-bicyclo[6.1.0]non-4-yn-9-ylmethyl N-succinimidyl carbonate (BCN-NHS) (93.0 mg) were added, and the mixture was stirred at room temperature for 2 hours. After concentration, purification was performed by reverse phase silica gel column chromatography (0.1% TFA aqueous solution/MeOH = 90/10 to 30/70) to obtain ((1R,8S,9s)-bicyclo[6.1.0]non-4-yn-9-yl)methyl(2-(2-(2-(3,5-bis(6-(5-((3aS,4S,6aR)-2-iminohexahydro-1H-thieno[3,4-d]imidazol-4-yl)pentanamido)hexanamido)benzamido)ethoxy)ethoxy)ethyl)car bamate di(2,2,2-trifluoroacetate) (compound (9)) (302 mg).

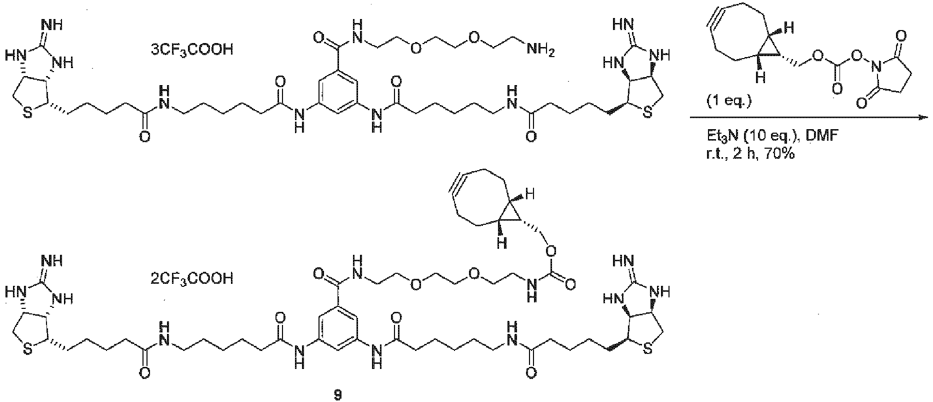

¹H NMR(400MHz,CD₃OD)δ:8.02(brs,1H),7.72(d,J=1.6Hz,2H),4.71(dd, J=4.4,7.6Hz,2H),4.52(dd,J=4.4,7.6Hz,2H),4.09(d,J=8.4Hz,2H), 3.70-3.62(m,6H),3.59-3.52(m,4H),3.29-3.24(m,4H),3.19(t,J=7.2Hz,4H),2.98(dd,J=4.8,13.2Hz,2H),2.81 (d,J=13.2Hz,2H),2.40(t,J=7.2Hz,4H),2.26-2.10(m, 10H), 1.79-1.51 (m,18H),1.47-1.38(m,8H),1.38-1.30(m,1H),0.920(t,J=9.6Hz,2H)

### [Example 75]

### Production of compound (9)-bonded SCNP

In a glass vial, N₃-poly[(benzyl acrylate)-co-(poly(ethylene glycol) methyl ether acrylate)-co-(acrylic acid)] (200 mg) and the compound (9) obtained in Example 74 (30 mg) were dissolved in DMF (5 mL), and the mixture was stirred at room temperature overnight. The reaction solution was dialyzed against methanol, the solvent was distilled off, and then the compound (9)-bonded SCNP (197.4 mg) was recovered by the vacuum drying. A Z-average particle diameter and a polydispersity index of the compound (9)-bonded SCNP were measured by dynamic light scattering (DLS), and a result of the Z-average particle diameter was 7.2 nm (polydispersity index: 0.18).

### [Measurement apparatuses and conditions]

### (1) ¹H-NMR measurement

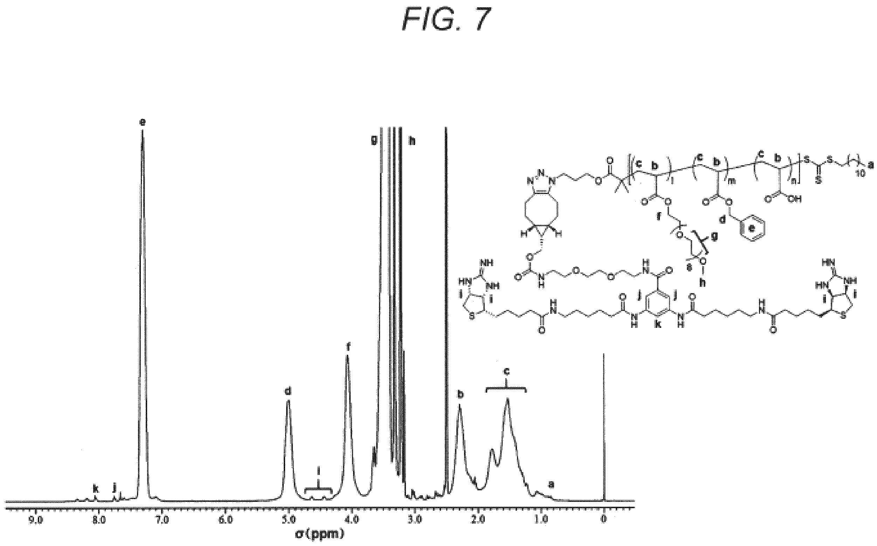

Apparatus: JNM-ECX400 (400 MHz)/JEOL Ltd.
Solvent: dimethyl sulfoxide-d₆ containing 0.03% tetramethylsilane/KANTO CHEMICAL CO., INC.
Sample concentration: 20 mg/mL
Measurement temperature: 25°C
Number of integration times: 6 000 times
Results: Fig. 7

### (2) GPC measurement

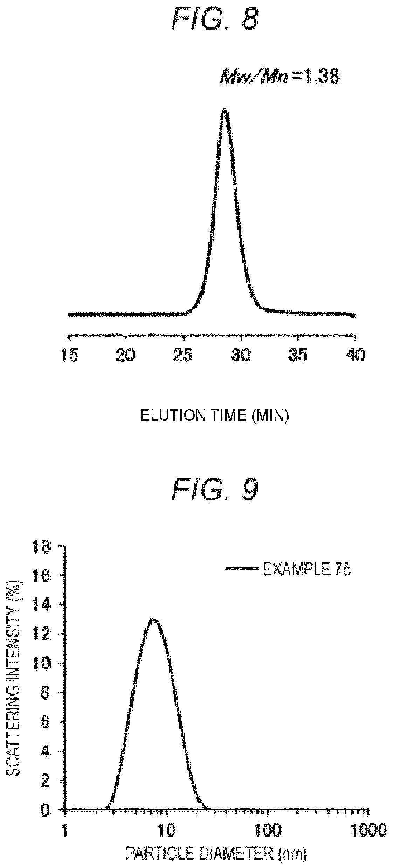

Apparatus: HPLC-Prominence system/SHIMADZU CORPORATION
Detector: RID-10A Refractive index detector/SHIMADZU CORPORATION
Column: TSKgel α-2500 column/Tosoh Corporation
(Column size: 7.8 mm × 300 mm, particle size: 7 µm,
and exclusion limit molecular weight: 5 × 10³)
TSKgel α-4000 column/Tosoh Corporation
(Column size: 7.8 mm × 300 mm, particle size: 10 µm, and exclusion limit molecular weight: 4 × 10⁵)
TSKgel guard column/Tosoh Corporation
Mobile phase: N,N-dimethyformamide (DMF) containing 10 mmol/L of lithium bromide
Temperature: 40°C
Flow rate: 0.5 mL/min
Sample concentration: 6 mg/mL
Standard substance: poly(methyl methacrylate) standard ReadyCal set, Mₚ 800 - 2 200 000 Da/SIGMA
Results: Fig. 8

### (3) DLS measurement

Apparatus: Zetasizer NanoZS/Malvern Instruments Ltd.
Measurement temperature: 25°C
Sample concentration: 10 mg/mL
Results: Fig. 9

### [Example 76]

### Synthesis of ((1R,8S,9s)-bicyclo[6.1.0]non-4-yn-9-yl)methyl(2-(2-(2-(3-(3,5-bis((2-(2-(5-((3aS,4S,6aR)-2-iminohexahydro-1H-thieno[3,4-d]imidazol-4-yl)pentanamido)ethoxy)ethyl)carbamoyl)phenyl)ureido)ethoxy) ethoxy)ethyl)carbamate di(2,2,2-trifluoroacetate)

To 5-(3-(2-(2-(2-aminoethoxy)ethoxy)ethyl)ureido)-N¹,N³-bis(2-(2-(5-((3aS,3a'S,4S,4'S,6aR,6a'R)-2-iminohexahydro-1H-thieno[3,4-d]imidazol-4-yl)pentanamide)ethoxy)ethyl)isophthalamide tri(2,2,2-trifluoroacetate) (450 mg), DMF (15 mL), Et₃N (475 µL), and BCN-NHS (99.3 mg) were added, and the mixture was stirred at room temperature for 2 hours. After concentration, purification was performed by reverse phase silica gel column chromatography (0.1% TFA aqueous solution/MeOH = 90/10 to 30/70) to obtain ((1R,8S,9s)-bicyclo[6.1.0]non-4-yn-9-yl)methyl(2-(2-(2-(3-(3,5-bis((2-(2-(5-((3aS,4S,6aR)-2-iminohexahydro-1H-thieno[3,4-d]imidazol-4-yl)pentanamido)ethoxy)ethyl)carbamoyl)phenyl)ureido)ethoxy) ethoxy)ethyl)carbamate di(2,2,2-trifluoroacetate) (compound (10)) (327 mg).

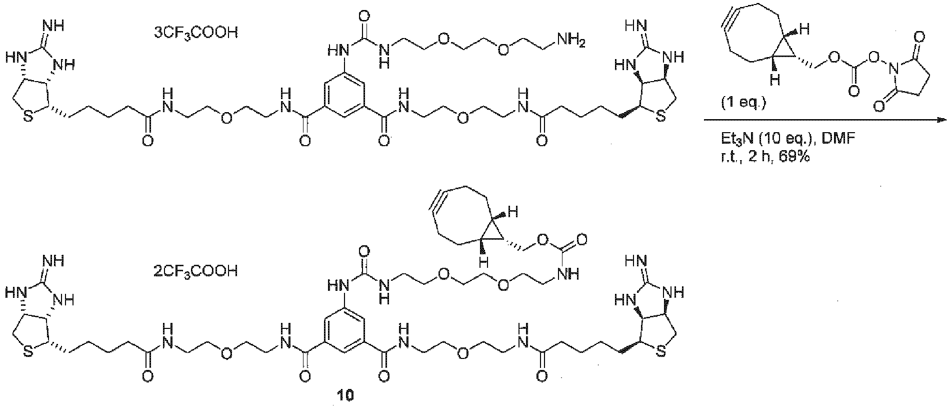

¹H NMR(400MHz,CD₃OD)δ:8.00(d,J=1.2Hz,2H),7.88(t,J=1.6Hz,1H),4. 71(dd, J=4.4,7.6Hz,2H),4.50(dd, J=4.0,7.6Hz,2H),4.17(d, J=8.4H z,2H),3.67-3.63(m,8H),3.62-3.51 (m,12H),3.42(t,J=5.2Hz,2H),3.38(t,J=5.2Hz,4H),3.29-3.27 (m,2H),3.26-3.19(m, 2H), 2.98(dd,J=4.8,12.8Hz,2H),2.81(d,J=12.8Hz,2H),2.2 6-2.10(m,10H),1.73-1.62(m,2H),1.61-1.46(m,8H),1.41-1.28(m,5H),0.90(t,J=9.6Hz,2H)

### [Example 77]

### Production of compound (10)-bonded SCNP

In a glass vial, N₃-poly[(benzyl acrylate)-co-(poly(ethylene glycol) methyl ether acrylate)-co-(acrylic acid)] (200 mg) and the compound (10) obtained in Example 76 (30 mg) were dissolved in DMF (5 mL), and the mixture was stirred at room temperature overnight. The reaction solution was dialyzed against methanol, the solvent was distilled off, and then the compound (10)-bonded SCNP (195.8 mg) was recovered by the vacuum drying. A Z-average particle diameter and a polydispersity index of the compound (10)-bonded SCNP were measured by dynamic light scattering (DLS), and a result of the Z-average particle diameter was 8.3 nm (polydispersity index: 0.19).

### [Measurement apparatuses and conditions]

### (1) ¹H-NMR measurement

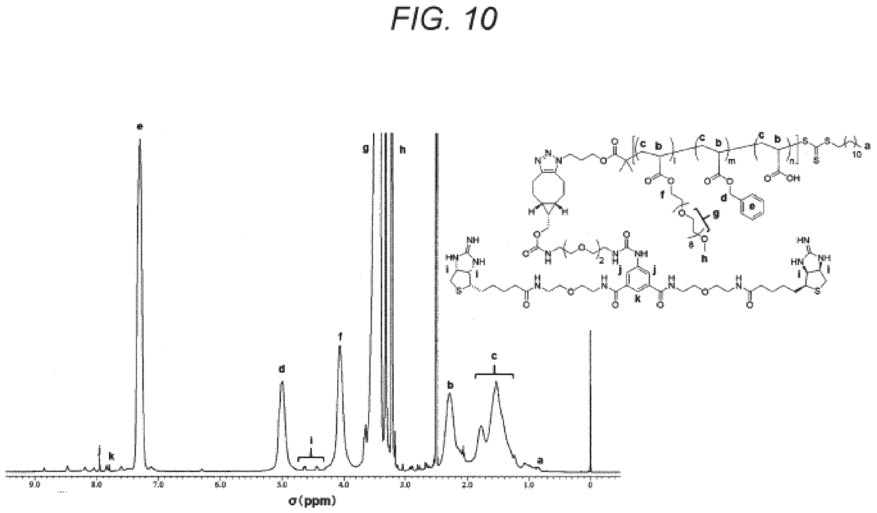

Apparatus: JNM-ECX400 (400 MHz)/JEOL Ltd.
Solvent: dimethyl sulfoxide-d₆ containing 0.03% tetramethylsilane/KANTO CHEMICAL CO., INC.
Sample concentration: 20 mg/mL
Measurement temperature: 25°C
Number of integration times: 6 000 times
Results: Fig. 10

### (2) GPC measurement

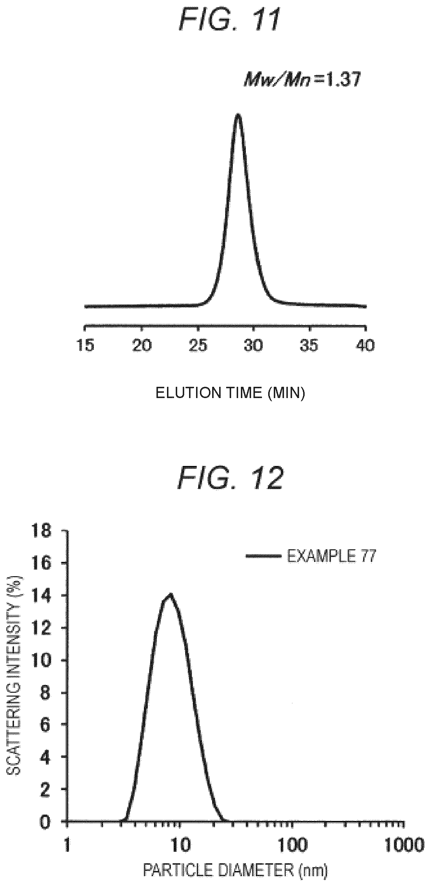

Apparatus: HPLC-Prominence system/SHIMADZU CORPORATION
Detector: RID-10A Refractive index detector/SHIMADZU CORPORATION
Column: TSKgel α-2500 column/Tosoh Corporation
(Column size: 7.8 mm × 300 mm, particle size: 7 µm, and exclusion limit molecular weight: 5 × 10³)
TSKgel α-4000 column/Tosoh Corporation
(Column size: 7.8 mm × 300 mm, particle size: 10 µm, and exclusion limit molecular weight: 4 × 10⁵)
TSKgel guard column/Tosoh Corporation
Mobile phase: N,N-dimethy formamide (DMF) containing 10 mmol/L of lithium bromide
Temperature: 40°C
Flow rate: 0.5 mL/min
Sample concentration: 6 mg/mL
Standard substance: poly(methyl methacrylate) standard ReadyCal set, Mₚ 800 to 2 200 000 Da/SIGMA
Results: Fig. 11

### (3) DLS measurement

Apparatus: Zetasizer NanoZS/Malvern Instruments Ltd.
Measurement temperature: 25°C
Sample concentration: 10 mg/mL
Results: Fig. 12

### [Example 78]

### Synthesis of dimethyl 6,6'-((5-(3-(1-((1R,8S,9s)-bicyclo[6.1.0]non-4-yn-9-yl)-3-oxo-2,7,10-trioxa-4-azadodecane-12-yl)ureido)isophthaloyl)bis(azanediyl)) (2S,2'S)-bis(2-(5-((3aS,4S,6aR)-2-iminohexahydro-1H-thieno[3,4-d]imidazol-4-yl)pentamido)hexanoate) di(2,2,2-trifluoroacetate)

To (R,S,S,2S,2'S)-dimethyl 6,6'-((5-(3-(2-(2-(2-aminoethoxy)ethoxy)ethyl)ureido)isophthaloyl)bis(azanediyl) )bis(2-(5-((3aS,4S,6aR)-2-iminohexahydro-1H-thieno[3,4-d]imidazol-4-yl)pentanamide)hexanoate) tri(2,2,2-trifluoroacetate) (165 mg), DMF (10 mL), Et₃N (160 µL), and BCN-NHS (33.6 mg) were added, and the mixture was stirred at room temperature for 2 hours. After concentration, purification was performed by reverse phase silica gel column chromatography (0.1% TFA aqueous solution/MeOH = 90/10 to 30/70) to obtain dimethyl 6,6'-((5-(3-(1-((1R,8S,9s)-bicyclo[6.1.0]non-4-yn-9-yl)-3-oxo-2,7,10-trioxa-4-azadodecane-12-yl)ureido)isophthaloyl)bis(azanediyl))(2S,2'S)-bis(2-(5-((3aS,4S,6aR)-2-iminohexahydro-1H-thieno[3,4-d]imidazol-4-yl)pentamido)hexanoate) di(2,2,2-trifluoroacetate) (compound (11)) (120 mg).

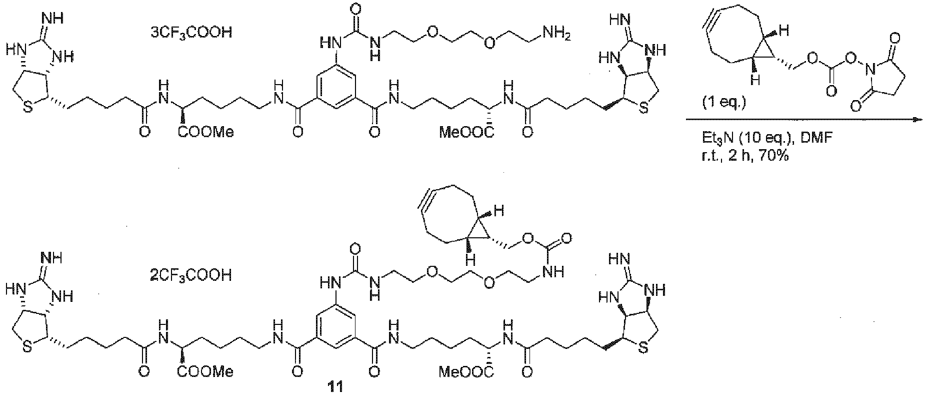

¹H NMR(400MHz,CD₃OD)δ:7.95(d,J=1.6Hz,2H),7.81(t,J=1.6Hz,1H),4. 72(dd,J=4.4,7.6Hz,2H),4.52(dd,J=4.4,7.6Hz,2H),4.41(dd,J=4.8 ,9.6Hz,2H),4.11(d,J=7.6Hz,2H),3.71(s,6H),3.65(s,4H),3.60(t, J=5.6Hz,2H),3.55(t,J=5.6Hz,2H),3.44-3.37(m,6H),3.29-3.25(m,4H),2.99(dd,J=4.8,12.8Hz,2H),2.81(d,J=13.2Hz,2H),2.2 8-2.10(m,10H),1.94-1.84(m,2H),1.79-1.32(m,25H),0.90(t,J=9.6Hz,2H)

### [Example 79]

### Production of compound (11)-bonded SCNP

In a glass vial, N₃-poly[(benzyl acrylate)-co-(poly(ethylene glycol) methyl ether acrylate)-co-(acrylic acid)] (200 mg) and the compound (11) obtained in Example 78 (30 mg) were dissolved in DMF (5 mL), and the mixture was stirred at room temperature overnight. The reaction solution was dialyzed against methanol, the solvent was distilled off, and then the compound (11)-bonded SCNP (198.5 mg) was recovered by the vacuum drying. A Z-average particle diameter and a polydispersity index of the compound (11)-bonded SCNP were measured by dynamic light scattering (DLS), and a result of the Z-average particle diameter was 8.6 nm (polydispersity index: 0.25).

### [Measurement apparatuses and conditions]

### (1) ¹H-NMR measurement

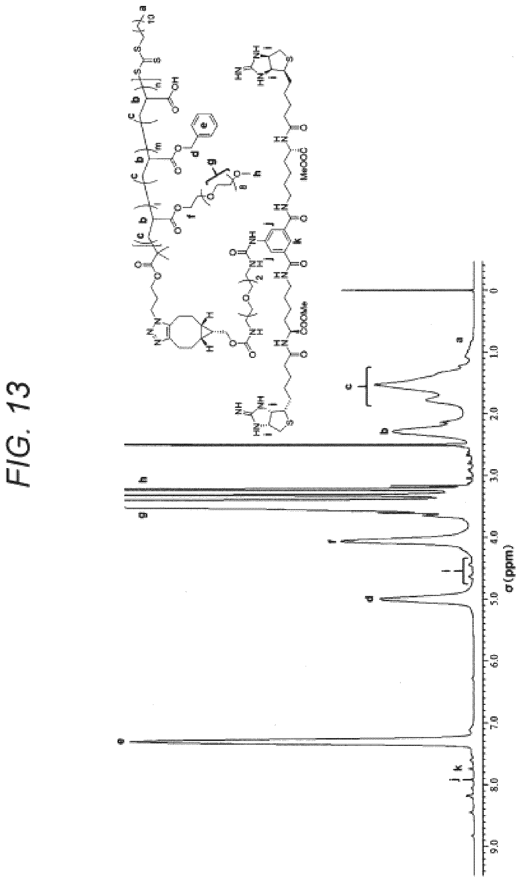

Apparatus: JNM-ECX400 (400 MHz)/JEOL Ltd.
Solvent: dimethyl sulfoxide-d₆ containing 0.03% tetramethylsilane/KANTO CHEMICAL CO., INC.
Sample concentration: 20 mg/mL
Measurement temperature: 25°C
Number of integration times: 6 000 times
Results: Fig. 13

### (2) GPC measurement

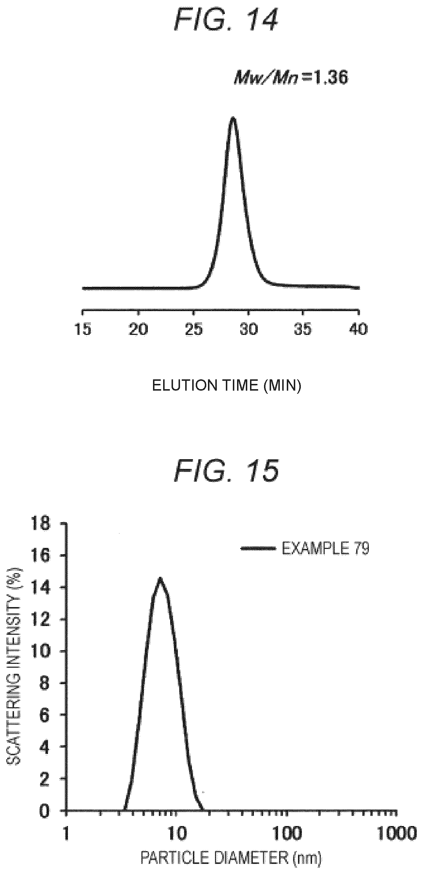

Apparatus: HPLC-Prominence system/SHIMADZU CORPORATION
Detector: RID-10A Refractive index detector/SHIMADZU CORPORATION
Column: TSKgel α-2500 column/Tosoh Corporation
(Column size: 7.8 mm × 300 mm, particle size: 7 µm, and exclusion limit molecular weight: 5 × 10³)
TSKgel α-4000 column/Tosoh Corporation
(Column size: 7.8 mm × 300 mm, particle size: 10 µm, and exclusion limit molecular weight: 4 × 10⁵)
TSKgel guard column/Tosoh Corporation
Mobile phase: N,N-dimethyformamide (DMF) containing 10 mmol/L of lithium bromide
Temperature: 40°C
Flow rate: 0.5 mL/min
Sample concentration: 6 mg/mL
Standard substance: poly(methyl methacrylate) standard ReadyCal set, Mₚ 800 - 2 200 000 Da/SIGMA
Results: Fig. 14

### (3) DLS measurement

Apparatus: Zetasizer NanoZS/Malvern Instruments Ltd.
Measurement temperature: 25°C
Sample concentration: 10 mg/mL
Results: Fig. 15

### [Comparative Example 1]

### Preparation of oxaliplatin solution

To 5.58 mL of a 5.9 (w/v)% glucose solution was added 1 mL of ELPLAT I.V. infusion solution 50 mg (Yakult Honsha Co., Ltd.) to prepare a 5 (w/v)% glucose solution containing 760 µg of oxaliplatin.

### [Test Example 1] Drug efficacy test

A tumor-bearing model obtained by subcutaneously transplanting a mouse colon cancer cell line C26 (American Type Culture Collection) into a female nude mouse (BALB/c-nu nu/nu, 7 weeks old; Charles River Laboratories Japan, Inc.) was used for a drug efficacy test.

A mouse colon cancer cell line C26 subcultured in a CO₂ incubator was suspended in a liquid medium (Dulbecco's Modified Eagle's Medium-high glucose, Sigma-Aldrich Co. LLC), and injected subcutaneously in the back of nude mice so that the number of cells per mouse was 1 × 10⁶/100 µL. Thereafter, the nude mice were raised for about 1 week, and administration of a drug was started when the average value of the tumor volume was grown to about 30 mm³. A DACHPt-encapsulating SCNP (DACHPt-encapsulating SCNP prepared using the copolymer of Example 70) was administered into the tail vein (3 times every other day), and the antitumor effect was evaluated from the tumor volume (4 to 5 mice in 1 group). As a comparison, an oxaliplatin solution (Comparative Example 1) was used, and the oxaliplatin solution was administered in the same manner. The dose of each preparation was 8 mg/kg (3.9 mg/kg in terms of Pt) as the maximum administrable dose for the oxaliplatin solution, and 3 mg/kg in terms of Pt for the DACHPt-encapsulating SCNP.

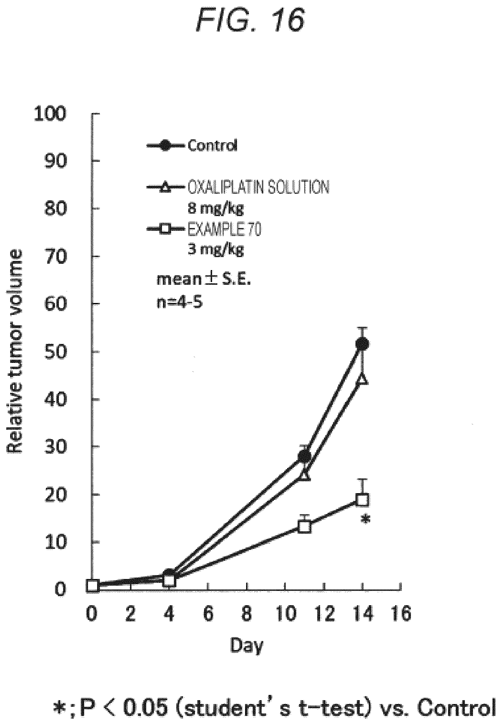

A change in the tumor volume over time is shown in Fig. 16. For the DACHPt-encapsulating SCNP, T/C was 0.4 after 14 days from administration [T/C: ratio of the tumor volume of the drug administration group (T) to that of the control group (C)]. For the oxaliplatin solution (Comparative Example 1), T/C was 1.1 after 14 days from administration. In addition, after 14 days from administration, it was confirmed that the DACHPt-encapsulating SCNP significantly suppressed growth of a tumor as compared with the control (student's t-test). The above results indicate that the DACHPt-encapsulating SCNP has an excellent antitumor effect as compared with the oxaliplatin solution.

## Claims

1. A copolymer in which a target-affinity molecule is bonded to a copolymer X comprising structural units represented by the following formulas (A), (B), and (C): 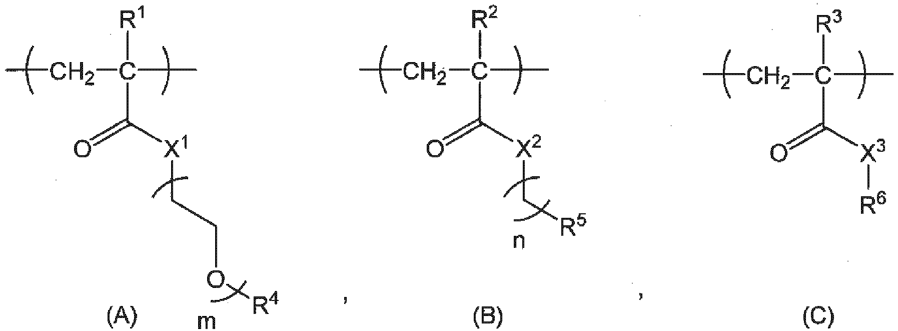 wherein, R¹, R², and R³ are the same or different and represent a hydrogen atom or a C₁₋₃ alkyl group, R⁴ represents a C₁₋₃ alkyl group, R⁵ represents a hydrogen atom, a C₁₋₁₈ alkyl group, a 3- to 8- membered cycloalkyl group optionally having a substituent, an adamantyl group, a C₆₋₁₈ aryl group optionally having a substituent, or a 5-to 10- membered heteroaryl group optionally having a substituent, X¹, X², and X³ are the same or different and represent an oxygen atom, a sulfur atom, or N-R⁷, R⁶ represents a hydrogen atom, a leaving group, or a linker, R⁷ represents a hydrogen atom or a C₁₋₃ alkyl group, m represents an integer of 1 to 100, and n represents an integer of 0 to 3.

2. The copolymer according to claim 1, wherein the polymer X is a copolymer formed by polymerization of three types of monomers represented by the following general formulas (1) to (3), 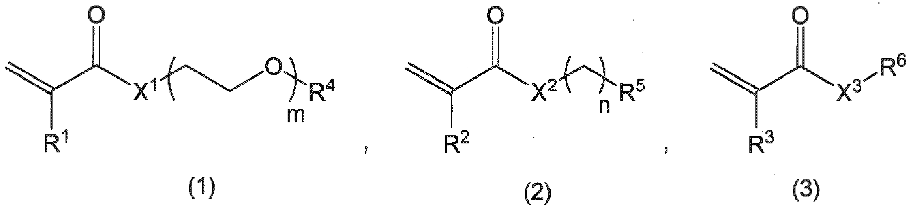 wherein, R¹, R², and R³ are the same or different and represent a hydrogen atom or a C₁₋₃ alkyl group, R⁴ represents a C₁₋₃ alkyl group, R⁵ represents a hydrogen atom, a C₁₋₁₈ alkyl group, a 3- to 8-membered cycloalkyl group optionally having a substituent, an adamantyl group, a C₆₋₁₈ aryl group optionally having a substituent, or a 5-to 10-membered heteroaryl group optionally having a substituent, X¹, X², and X³ are the same or different and represent an oxygen atom, a sulfur atom, or N-R⁷, R⁶ represents a hydrogen atom, a leaving group, or a linker, R⁷ represents a hydrogen atom or a C₁₋₃ alkyl group, m represents an integer of 1 to 100, and n represents an integer of 0 to 3.

3. The copolymer according to claim 1 or 2, wherein R¹ is a hydrogen atom.

4. The copolymer according to claim 1 or 2, wherein R² is a hydrogen atom.

5. The copolymer according to claim 1 or 2, wherein R³ is a hydrogen atom.

6. The copolymer according to claim 1 or 2, wherein R⁴ is a methyl group.

7. The copolymer according to claim 1 or 2, wherein R⁵ is a C₆₋₁₈ aryl group optionally having a substituent.

8. The copolymer according to claim 1 or 2, wherein R⁵ is a phenyl group.

9. The copolymer according to claim 1 or 2, wherein R⁶ is a hydrogen atom.

10. The copolymer according to claim 1 or 2, wherein the leaving group of R⁶ is a group represented by the following formula (4).

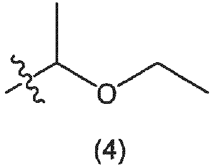

11. The copolymer according to claim 1 or 2, wherein the linker of R⁶ is one selected from the following formulas (5) to (7):

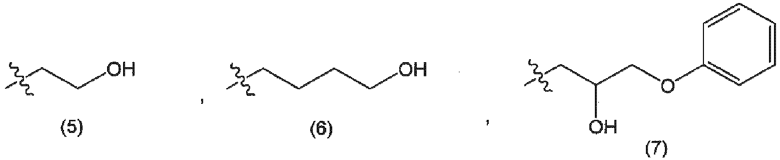

12. The copolymer according to claim 1 or 2, wherein X¹ is an oxygen atom.

13. The copolymer according to claim 1 or 2, wherein X² is an oxygen atom.

14. The copolymer according to claim 1 or 2, wherein X³ is an oxygen atom.

15. The copolymer according to claim 1 or 2, wherein m is an integer of 4 to 22.

16. The copolymer according to claim 1 or 2, wherein n is 1.

17. The copolymer according to claim 1 or 2, wherein a ratio of structural units (A), (B), and (C) is composed of 0.01 to 100 parts by mass of (B) and 0.1 to 100 parts by mass of (C) with respect to 1 part by mass of (A).

18. The copolymer according to claim 2, wherein 0.01 to 100 parts by mass of monomer (2) and 0.1 to 100 parts by mass of monomer (3) are polymerized with respect to 1 part by mass of monomer (1).

19. The copolymer according to claim 1 or 2, wherein the copolymer has a number average molecular weight of 5 000 to 150 000.

20. The copolymer according to claim 1 or 2, wherein the target-affinity molecule is biotin or a derivative thereof.

21. The copolymer according to claim 1 or 2, wherein the target-affinity molecule is a molecule having a biotin residue represented by the following formula (a): 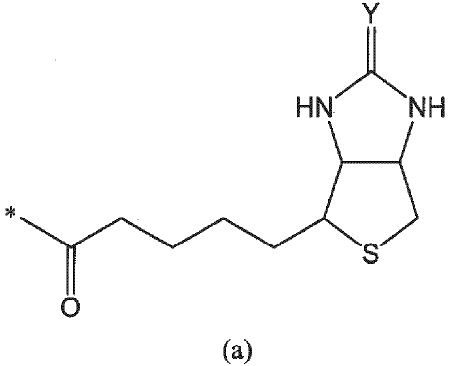 wherein, Y represents O or NH.

22. The copolymer according to claim 1 or 2, wherein the target-affinity molecule is any one of molecules represented by the following formulas (8) to (11).

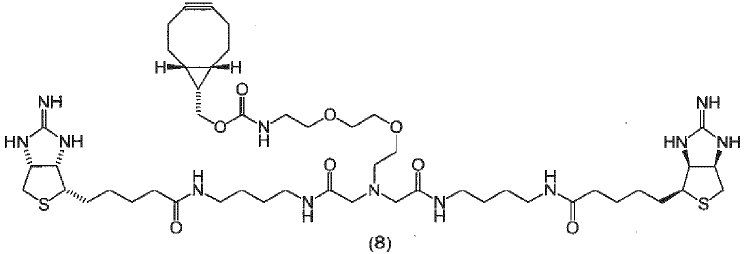

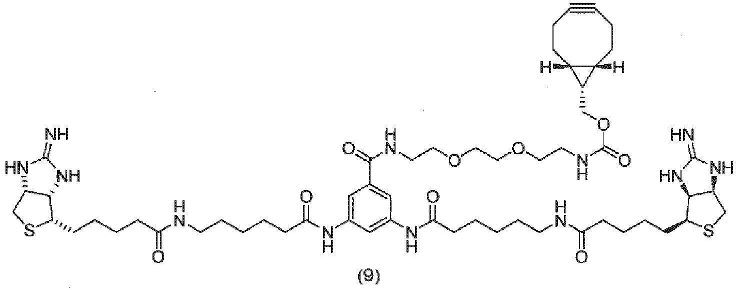

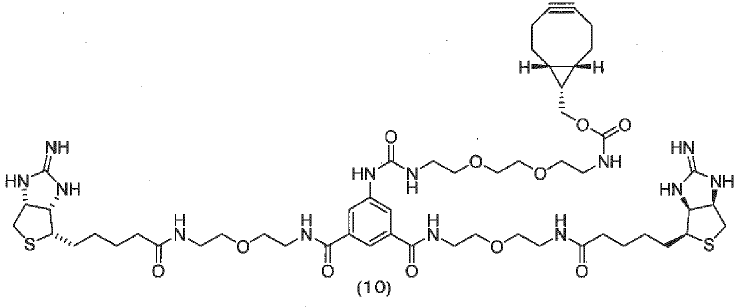

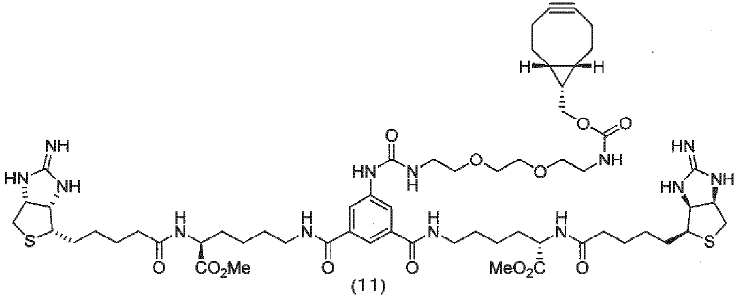

23. The copolymer according to claim 1 or 2, wherein the bond of the target-affinity molecule to the copolymer X is a covalent bond or a non-covalent bond.

24. A single chain nanoparticle comprising the copolymer according to claim 1 or 2.

25. A pharmaceutical composition comprising the copolymer according to claim 1 or 2.

26. The pharmaceutical composition according to claim 25, for use in a pre-targeting method.
